(19) Europäisches Patentamt
European Patent Office
Office européen des brevets

(11) Veröffentlichungsnummer: **0 003 056**
**B1**

(12) **EUROPÄISCHE PATENTSCHRIFT**

(45) Veröffentlichungstag der Patentschrift: **18.08.82**

(21) Anmeldenummer: **78101868.4**

(22) Anmeldetag: **29.12.78**

(51) Int. Cl.³: **C 07 C 103/76,** C 07 C 103/50, C 07 C 103/52, C 07 D 307/68, C 07 D 207/16, A 61 K 31/195, A 61 K 31/33

(54) **N-substituierte omega-Aminoalkanoyl-omega-aminoalkansäuren, ihre Verwendung und Verfahren zu ihrer Herstellung sowie diese Verbindungen enthaltende Arzneimittel.**

(30) Priorität: **30.12.77 LU 78804**

(43) Veröffentlichungstag der Anmeldung: **25.07.79 Patentblatt 79/15**

(45) Bekanntmachung des Hinweises auf die Patenterteilung: **18.08.82 Patentblatt 82/33**

(84) Benannte Vertragsstaaten: **BE CH DE FR GB IT LU NL SE**

(56) Entgegenhaltungen:
**DE-A-2 727 670**
**FR-A-2 236 868**
**FR-A-2 294 694**
**FR-A-2 377 374**

(73) Patentinhaber: **Byk Gulden Lomberg Chemische Fabrik GmbH, Byk-Gulden-Strasse 2, D-7750 Konstanz (DE)**

(72) Erfinder: **Krastinat, Walter, Dr., Erfurter Strasse 9, D-7750 Konstanz (DE)**
Erfinder: **Rapp, Erich, Dr., Irisweg 5, D-7760 Radolfzell 15 (DE)**
Erfinder: **Riedel, Richard, Dr., Bruelstrasse 22, D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 003 056 B1

N-substituierte omega-Aminoalkanoyl-omega-aminoalkansäuren, ihre Verwendung und Verfahren zu ihrer Herstellung sowie diese Verbindungen enthaltende Arzneimittel

Die Erfindung betrifft N-substituierte ω-Aminoalkanoyl-ω-aminoalkansäuren, ihre Verwendung und Verfahren zu ihrer Herstellung sowie diese Verbindungen enthaltende Arzneimittel.

In der deutschen Offenlegungsschrift DE-A-19 17 036 werden choleretisch wirkende N-Acyl-ω-anilinoalkansäuren beschrieben, denen auch noch weitere Wirkungen zugesprochen werden (DE-A-24 50 680). Trialkoxybenzoylpeptide (DE-A-23 38 172) eignen sich zur Prophylaxe und Behandlung von Herzkrankheiten. In der FR-A-2 294 694 werden Tyrosinderivate beschrieben, denen antiulcerogene Eigenschaften zugesprochen werden. In der DE-A-27 27 670 werden Dipeptide beschrieben, die als Elastasehemmer Verwendung finden sollen. Es wurde nun eine Klasse von ω-Aminoalkanoyl-ω-aminoalkansäuren synthetisiert, die weder in den erwähnten publizierten Patentanmeldungen genannt, noch durch sie nahegelegt wird. Ferner wurde gefunden, dass diese neuen Verbindungen interessante, besonders vorteilhafte pharmakologische Eigenschaften besitzen. So wurde u.a. überraschenderweise gefunden, dass sie eine Steigerung der Pankreassekretion herbeiführen, während die Verbindungen der DE-A-27 27 670 eine Hemmung der aus Pankreas gewinnbaren Elastase bewirken.

Gegenstand der Erfindung sind N-substituierte ω-Aminoalkanoyl-ω-aminoalkansäuren der allgemeinen Formel I

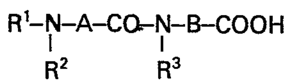 (I),

worin

$R^1$ einen Alkanoylrest oder Alkenoylrest mit 2 bis 5 Kohlenstoffatomen, einen Furoylrest oder einen Benzoylrest

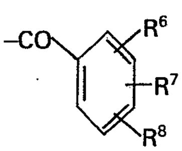

bedeutet,

$R^2$ ein Wasserstoffatom, den Rest $-C(R^9)(R^{10})(R^{11})$ oder einen Phenylrest

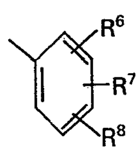

bedeutet,

$R^3$ ein Wasserstoffatom, einen Alkylrest mit 1 bis 5 Kohlenstoffatomen, den Rest $-C(R^9)(R^{10})(R^{11})$ oder einen Phenylrest

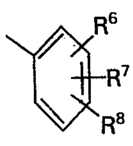

bedeutet,

wobei $R^2$ und $R^3$ nicht gleichzeitig ein Wasserstoffatom und nicht gleichzeitig einen geradkettigen Alkylrest darstellen,

A eine $-(CH_2)_m$-Gruppe oder eine $-CH(R^4)$-Gruppe bedeutet,

B eine $-(CH_2)_n$-Gruppe oder eine $-CH(R^5)$-Gruppe bedeutet,

m und n gleich oder verschieden sind und eine positive ganze Zahl von 1 bis 5 bedeuten,

$R^4$ und $R^5$ gleich oder verschieden sind und eine Methylgruppe, eine Benzylgruppe, eine Hydroxymethylgruppe, eine 2-Hydroxyethylgruppe, eine Methylmercaptomethylgruppe oder eine 2-Methylmercaptoethylgruppe bedeuten oder (unter der Voraussetzung, dass $R^2$ kein Wasserstoffatom darstellt)

$R^3$ und $R^5$ gemeinsam eine Trimethylgruppe bedeuten,

$R^6$, $R^7$ und $R^8$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

$R^9$ ein Wasserstoffatom, eine Alkylgruppe mit 1 bis 5 Kohlenstoffatomen, eine Alkenylgruppe oder eine Alkinylgruppe mit 2 bis 5 Kohlenstoffatomen,

$R^{10}$ ein Wasserstoffatom, eine Alkylgruppe mit 1 bis 5 Kohlenstoffatomen, eine Cycloalkylgruppe mit 3 bis 8 Kohlenstoffatomen oder einen Phenylrest

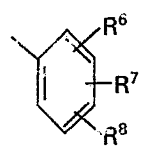

$R^{11}$ eine Alkylgruppe mit 1 bis 5 Kohlenstoffatomen, eine Cycloalkylgruppe mit 3 bis 8 Kohlenstoffatomen, eine Cycloalkylalkylgruppe mit 3 bis 8 Kohlenstoffatomen in der Cycloalkylgruppe und 1 bis 3 Kohlenstoffatomen in der Alkylgruppe, eine Phenylgruppe

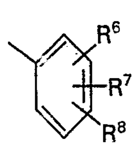

oder eine Phenylalkylgruppe mit 1 bis 3 Kohlenstoffatomen in der Alkylgruppe und mit dem Phenylrest

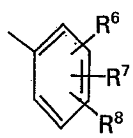

bedeutet oder

$R^{10}$ und $R^{11}$ gemeinsam eine Alkylengruppe mit 4 bis 7 Kohlenstoffatomen oder

$R^9$, $R^{10}$ und $R^{11}$ unter Einschluss des Kohlenstoffatoms, an das sie gebunden sind, einen Adamantylrest bedeuten,

sowie ihre Salze mit anorganischen und organischen Basen.

Alkanoyl- und Alkenoylreste mit 2 bis 5 Kohlenstoffatomen haben 1 bis 4 Kohlenstoffatome im Alkyl- bzw. Alkenylrest. Geradkettige Alkylreste sind der Methyl-, Ethyl-, Propyl- und Butylrest, von denen der Methyl- und Ethylrest bevorzugt ist. Verzweigte Alkylreste mit 3 oder 4 Kohlenstoffatomen sind der Isopropyl-, Isobutyl-, sek.-Butyl- oder ter.-Butylrest.

Von den Alkenylresten seien beispielsweise der Ethenyl- und insbesondere der 1-Propenylrest genannt.

Als Halogenatome $R^6$, $R^7$ und $R^8$ kommen Fluor, Chlor oder Brom, bevorzugt Fluor und Chlor, insbesondere Chlor, in Betracht.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemässen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niederalkyl-(z.B. Methyl) piperazinen, Methylcyclohexylamin, Benzylamin, Ethanolamin, Diethanolamin, Triethanolamin, Tris-(hydroxymethyl)-aminomethan, 2-Amino-2-methyl-propanol, 2-Amino-2-methyl-1,3-propandiol, Glucamin, N-Methylglucamin, Glucosamin, N-Methylglucosamin, Lysin, Ornithin, Arginin, Chinolin genannt.

Bei den Ausgestaltungen der Erfindung werden die Formeln, Substituenten und Symbole zur besseren Unterscheidung durch einen oder mehrere Sternchen gekennzeichnet.

Eine Ausgestaltung der Erfindung sind N-substituierte ω-Aminoalkanoyl-ω-aminoalkansäuren der allgemeinen Formel I *

$$\begin{array}{l} R^{1*}\text{–}\underset{\displaystyle R^{2*}}{\underset{|}{N}}\text{–}A^{*}\text{–CO–}\underset{\displaystyle R^{3*}}{\underset{|}{N}}\text{–}B^{*}\text{–COOH} \end{array} \qquad (I^*),$$

worin

$R^{1*}$ einen Alkanoylrest oder Alkenoylrest mit 2 bis 5 Kohlenstoffatomen, einen Furoylrest oder einen Benzoylrest

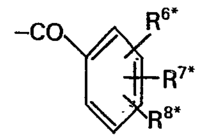

bedeutet,

$R^{2*}$ ein Wasserstoffatom, eine substituierte Niedrigalkylgruppe $-C(R^{9*})(R^{10*})(R^{11*})$ oder eine Phenylgruppe

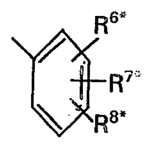

bedeutet,

$R^{3*}$ ein Wasserstoffatom oder eine gegebenenfalls substituierte Niedrigalkylgruppe bedeutet, wobei $R^{2*}$ und $R^{3*}$ nicht gleichzeitig ein Wasserstoffatom und $R^{2*}$ und $R^{3*}$ nicht gleichzeitig eine geradkettige Niedrigalkylgruppe darstellen,

$A^*$ eine $-(CH_2)_{m^*}$-Gruppe bedeutet,

$B^*$ eine $-(CH_2)_{n^*}$-Gruppe bedeutet,

$m^*$ und $n^*$ gleich oder verschieden sind und eine positive ganze Zahl von 1 bis 5 bedeuten,

$R^{6*}$, $R^{7*}$ und $R^{8*}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

$R^{9*}$ ein Wasserstoffatom, eine Alkylgruppe mit 1 bis 4 Kohlenstoffatomen oder eine Ethinylgruppe bedeutet,

$R^{10*}$ ein Wasserstoffatom, eine Alkylgruppe mit 1 bis 4 Kohlenstoffatomen oder einen Phenylrest

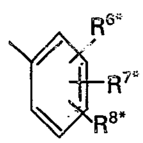

$R^{11*}$ eine Alkylgruppe mit 1 bis 5 Kohlenstoffatomen, einen Phenylrest

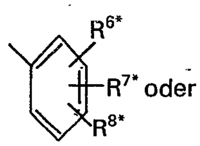

oder einen Benzylrest

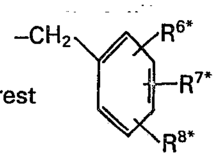

bedeutet oder

$R^{10*}$ und $R^{11*}$ gemeinsam eine Alkylengruppe mit 4 bis 7 Kohlenstoffatomen oder

$R^{9*}$, $R^{10*}$ und $R^{11*}$ unter Einschluss des benachbarten Kohlenstoffatoms einen Adamantyl-(1)-rest bedeuten,

und ihre Salze mit anorganischen und organischen Basen.

Bevorzugte Vertreter der Ausgestaltung I* sind solche, in denen $R^{1*}$ einen Alkanoylrest mit 2 bis 5 Kohlenstoffatomen oder einen durch $R^{6*}$, $R^{7*}$ und $R^{8*}$ substituierten Benzoylrest, $R^{2*}$ ein Wasserstoffatom, eine substituierte Niedrigalkylgruppe $-C(R^{9*})(R^{10*})(R^{11*})$ oder einen durch $R^{6*}$, $R^{7*}$ und $R^{8*}$ substituierten Phenylrest, $R^{3*}$ ein Wasserstoffatom, eine substituierte Niedrigalkylgruppe $-C(R^{9*})(R^{10*})(R^{11*})$ bedeuten, wobei $R^{2*}$ und $R^{3*}$ nicht gleichzeitig ein Wasserstoffatom und $R^{2*}$ und $R^{3*}$ nicht gleichzeitig eine geradkettige Niedrigalkylgruppe darstellen, A* eine $-(CH_2)_{m*}$-Gruppe und B* eine $-(CH_2)_{n*}$-Gruppe bedeuten, m* und n* gleich oder verschieden sind und eine positive ganze Zahl von 1 bis 5 bedeuten, $R^{6*}$ und $R^{7*}$ gleich oder verschieden sind und ein Wasserstoffatom, ein Halogenatom, eine Methylgruppe, eine Methoxygruppe, eine Aminogruppe, eine Nitrogruppe oder eine Trifluormethylgruppe darstellen, $R^{8*}$ ein Wasserstoffatom, $R^{9*}$ ein Wasserstoffatom oder eine Methylgruppe, $R^{10*}$ ein Wasserstoffatom oder einen durch $R^{6*}$, $R^{7*}$ und $R^{8*}$ substituierten Phenyl- oder einen durch $R^{6*}$, $R^{7*}$ und $R^{8*}$ substituierten Phenylrest, $R^{11*}$ einen durch $R^{6*}$, $R^{7*}$ und $R^{8*}$ substituierten Phenyl- oder einen durch $R^{6*}$, $R^{7*}$ und $R^{8*}$ substituierten Benzylrest bedeuten oder $R^{10*}$ und $R^{11*}$ gemeinsam eine Pentamethylengruppe oder eine Heptamethylengruppe darstellen, und ihre Salze mit anorganischen und organischen Basen.

Besonders bevorzugte Vertreter der Ausgestaltung I* sind solche, in denen $R^{1*}$ einen Alkanoylrest mit 2 bis 5 Kohlenstoffatomen oder einen durch $R^{6*}$, $R^{7*}$ und $R^{8*}$ substituierten Benzoylrest, $R^{2*}$ ein Wasserstoffatom oder eine durch $R^{6*}$, $R^{7*}$ und $R^{8*}$ substituierte Phenylgruppe, $R^{3*}$ ein Wasserstoffatom oder eine substituierte Niedrigalkylgruppe $-C(R^{9*})(R^{10*})(R^{11*})$ bedeuten, wobei $R^{2*}$ und $R^{3*}$ nicht gleichzeitig ein Wasserstoffatom darstellen, A* eine Trimethylengruppe, B* eine $-(CH_2)_{n*}$-Gruppe, n* eine positive ganze Zahl von 3 bis 5, $R^{6*}$ ein Wasserstoffatom, ein Chloratom, eine Methoxygruppe oder Trifluormethylgruppe, $R^{8*}$ ein Wasserstoffatom, $R^{9*}$ ein Wasserstoffatom, $R^{10*}$ eine durch $R^{6*}$, $R^{7*}$ und $R^{8*}$ substituierte Phenylgruppe, $R^{11*}$ eine durch $R^{6*}$, $R^{7*}$ und $R^{8*}$ substituierte Phenylgruppe oder eine durch $R^{6*}$, $R^{7*}$ und $R^{8**}$ substituierte Benzylgruppe bedeuten, und ihre Salze mit anorganischen und organischen Basen.

Eine andere Ausgestaltung der Erfindung sind N-substituierte ω-Aminoalkanoyl-ω-aminoalkansäuren der allgemeinen Formel I**

$$\begin{matrix} R^{1**}-N-A^{**}-CO-N-B^{**}-COOH \\ \;\;\;\;\;\;\;| \;\;\;\;\;\;\;\;\;\;\;\;\;\;\;\;\;\;\;\;\; | \\ R^{2**} \;\;\;\;\;\;\;\;\;\;\;\;\;\;\;\; R^{3**} \end{matrix} \qquad (I^{**}),$$

worin

$R^{1**}$ einen Alkanoylrest oder Alkenoylrest mit 2 bis 5 Kohlenstoffatomen, einen Furoylrest oder einen Benzoylrest

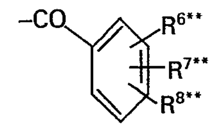

bedeutet,

$R^{2**}$ ein Wasserstoffatom oder eine Phenylgruppe

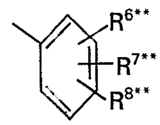

bedeutet,

$R^{3**}$ ein Wasserstoffatom oder eine Phenylgruppe

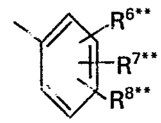

bedeutet, wobei $R^{2**}$ und $R^{3**}$ nicht gleichzeitig ein Wasserstoffatom darstellen,

A** eine $-(CH_2)_{m**}$-Gruppe bedeutet,

B** eine $-(CH_2)_{n**}$-Gruppe bedeutet,

m** und n** gleich oder verschieden sind und eine positive ganze Zahl von 1 bis 5 bedeuten,

$R^{6**}$, $R^{7**}$ und $R^{8**}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

und ihre Salze mit anorganischen und organischen Basen.

Bevorzugte Vertreter der Ausgestaltung I** sind solche, in denen $R^{1**}$ einen Alkanoylrest mit 2 bis 5 Kohlenstoffatomen oder einen durch $R^{6**}$, $R^{7**}$ und $R^{8**}$ substituierten Benzoylrest, $R^{2**}$ ein Wasserstoffatom oder einen durch $R^{6**}$, $R^{7**}$ und $R^{8**}$ substituierten Phenylrest, $R^{3**}$ ein Wasserstoffatom oder einen durch $R^{6**}$, $R^{7**}$ und $R^{8**}$ substituierten Phenylrest bedeuten, wobei $R^{2**}$ und $R^{3**}$ nicht gleichzeitig ein Wasserstoffatom darstellen, A** eine $-(CH_2)_{m**}$-Gruppe, B** eine $-(CH_2)_{n**}$-Gruppe, m** eine positive ganze Zahl von 1 bis 3, n** eine positive ganze Zahl von 3 bis 5 und $R^{6**}$ ein Wasserstoffatom bedeuten, $R^{7**}$ und $R^{8**}$ gleich oder verschieden sind und ein Wasserstoffatom, ein Halogenatom, eine Methylgruppe, eine Ethylgruppe, eine Methoxygruppe, eine Aminogruppe, eine Nitrogruppe oder eine Trifluormethylgruppe darstellen, und ihre Salze mit anorganischen und organischen Basen.

Besonders bevorzugte Vertreter der Ausgestaltung I** sind solche, in denen $R^{1**}$ einen Alkanoylrest mit 2 bis 5 Kohlenstoffatomen oder einen durch $R^{6**}$, $R^{7**}$ und $R^{8**}$ substituierten Benzoylrest, $R^{2**}$ einen durch $R^{6**}$, $R^{7**}$ und $R^{8**}$ substituierten Phenylrest, A** eine $-(CH_2)_{m^{**}}$-Gruppe, B** eine Trimethylengruppe, $m^{**}$ eine positive ganze Zahl von 1 bis 3, $R^{6**}$ ein Wasserstoffatom, $R^{7**}$ ein Wasserstoffatom, ein Chloratom, eine Methylgruppe, eine Methoxygruppe oder eine Trifluormethylgruppe, $R^{8**}$ ein Wasserstoffatom, ein Chloratom, eine Ethylgruppe, eine Methylgruppe oder eine Methoxygruppe bedeuten, und ihre Salze mit anorganischen und organischen Basen.

Eine weitere Ausgestaltung der Erfindung sind N-substituierte ω-Aminoalkanoyl-ω-aminoalkansäuren der allgemeinen Formel I***

$$R^{1***}-\underset{\displaystyle R^{2***}}{\underset{|}{N}}-A^{***}-CO-\underset{\displaystyle R^{3***}}{\underset{|}{N}}-B^{***}-COOH \qquad (I^{***}),$$

worin

$R^{1***}$ einen Alkanoyl- oder Alkenoylrest mit 2 bis 5 Kohlenstoffatomen, einen Furoylrest oder einen Benzoylrest

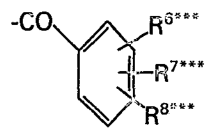

bedeutet,

$R^{2***}$ ein Wasserstoffatom oder einen Phenylrest

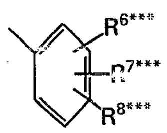

bedeutet,

$R^{3***}$ ein Wasserstoffatom, eine $-C(R^{9***})(R^{10***})(R^{11***})$-Gruppe oder eine durch $R^{6***}$, $R^{7***}$ und $R^{8***}$ substituierte Phenylgruppe bedeutet, wobei $R^{2***}$ und $R^{3***}$ nicht gleichzeitig ein Wasserstoffatom darstellen,

A*** eine $-(CH_2)_{m^{***}}$-Gruppe oder eine $-CH(R^{4***})$-Gruppe bedeutet,

B*** eine $-(CH_2)_{n^{***}}$-Gruppe oder eine $-CH(R^{5***})$-Gruppe bedeutet,

wobei

A*** und B*** nicht gleichzeitig eine geradkettige Alkylengruppe darstellen,

m*** und n*** gleich oder verschieden sind und eine positive ganze Zahl von 1 bis 5 bedeuten,

$R^{4***}$ und $R^{5***}$ gleich oder verschieden sind und eine Methylgruppe, eine Benzylgruppe, eine Hydroxymethylgruppe oder eine 2-Methylmercaptoethylgruppe bedeuten, oder (unter der Voraussetzung, dass $R^{2***}$ kein Wasserstoffatom darstellt)

$R^{3***}$ und $R^{5***}$ gemeinsam eine Trimethylengruppe bedeuten,

$R^{6***}$, $R^{7***}$ und $R^{8***}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoylgruppe mit 2 bis 5 Kohlenstoffatomen, eine Aminogruppe, eine Nitrogruppe oder eine Trifluormethylgruppe bedeuten,

$R^{9***}$ ein Wasserstoffatom, eine Alkylgruppe mit 1 bis 3 Kohlenstoffatomen oder eine Ethinylgruppe bedeutet,

$R^{10***}$ ein Wasserstoffatom, eine Alkylgruppe mit 1 bis 3 Kohlenstoffatomen, eine Cycloalkylgruppe mit 3 bis 8 Kohlenstoffatomen oder einen durch $R^{6***}$, $R^{7***}$ und $R^{8***}$ substituierten Phenylrest bedeutet,

$R^{11***}$ eine Alkylgruppe mit 1 bis 3 Kohlenstoffatomen, eine Cycloalkylgruppe mit 3 bis 8 Kohlenstoffatomen, eine durch $R^{6***}$, $R^{7***}$ und $R^{8***}$ substituierte Phenylgruppe, eine durch $R^{6***}$, $R^{7***}$ und $R^{8***}$ substituierte Benzylgruppe bedeutet, oder

$R^{10***}$ und $R^{11***}$ gemeinsam eine Alkylengruppe mit 4 bis 7 Kohlenstoffatomen bedeuten,

und ihre Salze mit anorganischen und organischen Basen.

Bevorzugte Vertreter der Ausgestaltung I*** sind solche, in denen $R^{1***}$ einen Alkanoylrest mit 2 bis 5 Kohlenstoffatomen oder einen durch $R^{6***}$, $R^{7***}$ und $R^{8***}$ substituierten Benzoylrest, $R^{2***}$ ein Wasserstoffatom oder einen durch $R^{6***}$, $R^{7***}$ und $R^{8***}$ substituierten Phenylrest, $R^{3***}$ einen durch $R^{6***}$, $R^{7***}$ und $R^{8***}$ substituierten Phenylrest, A*** eine $-(CH_2)_{m^{***}}$-Gruppe oder eine $-CH(R^{4***})$-Gruppe, B*** eine $-(CH_2)_{n^{***}}$-Gruppe oder eine $-CH(R^{5***})$-Gruppe bedeuten, wobei entweder A*** eine $-(CH_2)_{m^{***}}$-Gruppe oder B*** eine $-(CH_2)_{n^{***}}$-Gruppe darstellt, m*** und n*** gleich oder verschieden sind und eine positive ganze Zahl von 3 bis 5 darstellen, $R^{4***}$ und $R^{5***}$ gleich oder verschieden sind und eine Methylgruppe, eine Benzylgruppe, eine Hydroxymethylgruppe oder eine 2-Methylmercaptoethylgruppe bedeuten, oder (unter der Voraussetzung, das $R^{2***}$ kein Wasserstoffatom darstellt) $R^{3***}$ und $R^{5***}$ gemeinsam eine Trimethylengruppe bedeuten, $R^{6***}$ ein Wasserstoffatom bedeutet, $R^{7***}$ und $R^{8***}$ gleich oder verschieden sind und ein Wasserstoffatom, ein Halogenatom, eine Methylgruppe, eine Methoxygruppe, eine Nitrogruppe, eine Aminogruppe oder eine Trifluormethylgruppe darstellen, und ihre Salze mit anorganischen und organischen Basen.

Besonders bevorzugte Vertreter der Ausgestaltung I*** sind solche, in denen $R^{1***}$ einen Alkanoylrest mit 2 bis 5 Kohlenstoffatomen oder einen durch $R^{6***}$, $R^{7***}$ und $R^{8***}$ substituierten Benzoylrest bedeutet, $R^{2***}$ ein Wasserstoffatom oder einen durch $R^{6***}$, $R^{7***}$ und $R^{8***}$ substituierten Phenylrest bedeutet, $R^{3***}$ einen durch $R^{6***}$, $R^{7***}$ und $R^{8***}$ substituierten Phenylrest bedeutet, $R^{3***}$ einen durch $R^{6***}$, $R^{7***}$ und $R^{8***}$ substituierten Phenylrest bedeutet, A*** eine $-CH(R^{4***})$-Gruppe bedeutet, B*** eine Trimethylengruppe bedeutet,

$R^{4***}$ eine Methylgruppe, eine Benzylgruppe oder eine 2-Methylmercaptoethylgruppe bedeutet, $R^{6***}$ ein Wasserstoffatom, $R^{7***}$ ein Wasserstoffatom, ein Chloratom, eine Methyoxygruppe, eine Methylgruppe oder eine Trifluormethylgruppe, $R^{8***}$ ein Wasserstoffatom, ein Chloratom, eine Methylgruppe oder eine Methoxygruppe bedeuten, und ihre Salze mit anorganischen und organischen Basen.

Die Vertreter der Ausgestaltungen I** und I*** sind gegenüber denen der Ausgestaltung I* bevorzugt.

Als Vertreter der erfindungsgemässen Verbindungen seien genannt:

N-[N-p-Toluoyl-4-(m-trifluormethylanilino)-butyryl]-4-(2,6-dimethylanilino)-buttersäure,
N-[N-p-Toluoyl-3-(m-trifluormethylanilino)-propionyl]-4-(2,3-dimethylanilino)-buttersäure,
N-[N-3,4-Dimethylbenzoyl-2-(m-trifluormethylanilino)-propionyl]-3-(2,6-dimethylanilino)-propionsäure,
N-[N-p-Toluoyl-4-(o-anisidino)-butyryl]-4-(p-anisidino)-buttersäure,
N-[N-3,4-Dimethylbenzoyl-3-(p-anisidino)-propionyl]-4-(p-phenetidino)-buttersäure,
N-[N-3,4-Dimethoxybenzoyl-4-(2,6-dimethylanilino)-butyryl]-4-(2,6-dimethylanilino]-buttersäure,
N-[N-(2-Methoxy-5-chlor-benzoyl)-4-(p-anisidino)-butyryl]-4-(p-anisidino)-buttersäure,
N-[N-(2-Methoxy-5-chlor-benzoyl)-3-(p-anisidino)-propionyl]-3-(2,6-dimethylanilino)-propionsäure,
N-[N-(o-Chlorbenzoyl)-4-(p-phenetidino)-butyryl]-4-(2,6-dimethylanilino)-buttersäure,
N-[N-(o-Chlorbenzoyl)-3-(2,6-dimethylanilino)-propionyl]-4-(phenetidino)-buttersäure,
N-[N-(o-Chlorbenzoyl)-3-(p-phenetidino)-propionyl]-2-(p-phenetidino)-propionsäure,
N-[N-(p-Fluorbenzoyl)-2-(2,6-dimethylanilino)-acetyl]-4-(p-anisidino)-buttersäure,
N-[N-(o-Fluorbenzoyl)-3-(2-chlor-4-methylanilino)-propionyl]-4-(2-chlor-4-methylanilino)-buttersäure,
N-[N-(3,5-Dichlorbenzoyl)-3-(3,4-diethoxyanilino)-propionyl]-2-(3,4-diethoxyanilino-propionsäure,
N-[N-(p-Acetamido-benzoyl)-4-(p-chloranilino)-butyryl]-4-(2,4-dimethoxyanilino)-buttersäure,
N-[N-(α,α,α-Trifluor-m-toluoyl)-glycyl]-4-(2,6-dimethylanilino)-buttersäure,
N-[N-(2,4-Dichlorbenzoyl)-α-alanyl]-4-(p-anisidino)-buttersäure,
N-[N-(p-Nitrobenzoyl)-2-(o-toluidino)-propionyl]-4-(o-toluidino)-buttersäure,
N-[N-(o-Nitrobenzoyl)-3-(p-toluidino)-propionyl]-glycin,
N-[N-p-chlorbenzoyl-4-(4-methyl-3-nitro-anilino)-butyryl]-4-(3,4-dimethylanilino)-buttersäure,
N-[N-Benzoyl-(4-isopropylanilino)-acetyl]-4-(4-methoxy-2-methylanilino)-buttersäure,
N-[N-(o-Acetoxybenzoyl)-2-(p-anisidino)-acetyl]-L-prolin,
N-[N-Benzoyl-3-(3,4-dimethoxyanilino)-propionyl]-L-prolin,
N-[N-(p-Anisoyl)-2-(p-anisidino)-acetyl-L-prolin,
N-[N-(p-Chlorbenzoyl)-4-(p-anisidino)-butyryl]-4-benzhydrylamino-buttersäure,
N-[N-(p-Chlorbenzoyl)-3-(2,6-dimethylanilino)-propionyl]-3-benzhydrylamino-propionsäure,
N-[N-Propionyl-4-(2,6-dimethylanilino)-butyryl]-4-(2,6-dimethylanilino)-buttersäure
N-[N-Isobutyryl-3-(p-anisidino)-propionyl]-4-(p-anisidino)-buttersäure,
N-[N-Crotonyl-4-(2,6-diethylanilino)-butyryl]-4-(2,6-diethylanilino)-buttersäure,
N-[N-Crotonyl-α-alanyl]-4-(2-chlor-6-methyl-anilino)-buttersäure,
N-[N-(p-Chlorbenzoyl)-3-(p-anisidino)-propionyl]-5-(p-anisidino)-valeriansäure,
N-[N-(2-Methoxy-5-chlor-benzoyl)-4-(2,6-dimethylanilino)-butyryl]-5-(2,6-dimethylanilino)-valeriansäure,
N-[N-Acetyl-4-(p-anisidino)-butyryl]-6-benzhydrylamino-hexansäure,
N-[N-(2,4-Dichlorbenzoyl)-6-(p-phenetidino)-hexanoyl]-6-(p-phenetidino)-hexansäure,
N-[N-(p-Chlorbenzoyl)-4-(p-anisidino)-butyryl]-6-(2,6-dimethylanilino)-hexansäure,
und ihre Salze mit anorganischen und organischen Basen.

Als bevorzugte Einzelvertreter der erfindungsgemässen Verbindungen seien folgende Verbindungen und ihre pharmakologisch verträglichen Salze mit anorganischen und organischen Basen genannt:

N-[N-Acetyl-2-(p-anisidino)-acetyl]-4-(p-anisidino)-buttersäure,
N-[N-p-Chlorbenzoyl-4-(2,6-dimethylanilino)-butyryl]-4-(p-anisidino)-buttersäure,
N-[N-p-Chlorbenzoyl-4-(2,6-dimethylanilino)-butyryl]-4-aminobuttersäure,
N-[N-(3,4,5-Trimethoxybenzoyl)-4-(2,6-dimethylanilino)-butyryl]-4-amino-buttersäure,
N-[N-Acetyl-3-(2,6-dimethylanilino)-propionyl]-4-(2-ethyl-6-methyl-anilino)-buttersäure,
N-[N-p-Chlorbenzoyl-methionyl]-4-(p-anisidino)-buttersäure.

Erfindungsgemässe Verbindungen, die ein oder mehrere asymmetrische Kohlenstoffatome (Chiralitätszentren) enthalten, z. B. solche, in denen eine $-CH(R^4)$-Gruppe, $-CH(R^5)$-Gruppe und/oder $-C(R^9)(R^{10})(R^{11})$-Gruppe, in der $R^9$, $R^{10}$ und $R^{11}$ verschieden sind, vorliegen, fallen bei Herstellung aus racemischen Ausgangsstoffen in Form der Racemate bzw. Diastereomerengemische an, die in bekannter Weise, z.B. mit Hilfe optisch aktiver Basen bzw. aufgrund der Unterschiede ihrer physikochemischen Eigenschaften in die Enantiomeren aufgetrennt werden. Bei Einsatz optisch aktiver Verbindungen als Ausgangsstoffe werden die Endprodukte in optisch aktiver Form erhalten.

Die erfindungsgemässen Verbindungen weisen wertvolle Eigenschaften auf, die sie gewerblich verwertbar machen. Sie entfalten an Warmblütlern eine Schutzwirkung für Magen und Leber, daneben bewirken sie eine Steigerung der Sekretion von Pakreas und Leber (Galle).

Aufgrund ihrer vorteilhaften Wirksamkeit sind die erfindungsgemässen Verbindungen zur Behandlung und Prophylaxe von Krankheiten, die auf Erkrankungen des Magens oder Darms oder auf Minderleistungen von Pankreas, Galle und/

oder Leber beruhen, geeignet. Beispielsweise werden behandelt Magen- oder Darmulcera, Billroth II, Pankreas-Insuffizienz, Sprue, Maldigestionen und Malabsorptionen verschiedener Genese, akute und chronische Pankreatitis, indirekte Störungen der Pankreasfunktion (Unterstützung der Sekretin- und Pankreozymin-Produktion), daneben Gallenblasen- und Gallenwegsentzündungen, Gallenflussstörungen, Motilitätsstörungen der Gallenwege, Völlegefühl, Blähungen, Obstipationen, Oberbauchbeschwerden, hepatobiläre Funktionsstörungen, akute und chronische Hepatitis, Leberintoxikationen, Fettleber.

Ein weiterer Gegenstand der Erfindung sind Arzneimittel, die eine oder mehrere der N-substituierten ω-Aminoalkanoyl-ω-aminoalkansäuren der allgemeinen Formel I und/oder ihre pharmakologisch verträglichen Salze mit anorganischen oder organischen Basen erhalten.

Ausgestaltungen der Arzneimittel sind solche, die N-substituierte ω-Aminoalkanoyl-ω-aminoalkansäuren der Formeln I*, I**, I*** oder ihre bevorzugten Vertreter und/oder ihre pharmakologisch verträglichen Salze mit anorganischen oder organischen Basen enthalten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

Die erfindungsgemässen Arzneimittel können im human- und veterinärmedizinischen Bereich in jeder beliebigen Form, z.B. systemisch, angewandt werden unter der Voraussetzung, dass die Ausbildung bzw. Aufrechterhaltung von ausreichenden Blut- oder Gewebsspiegeln oder Lokalkonzentrationen an Wirkstoffen gewährleistet ist. Das kann entweder durch orale, rektale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion, einer Suspension, eines Sols oder eines Gels sein.

Unter «Einheitsdosis» wird in diesem Zusammenhang eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die erfindungsgemässen Arzneimittel enthalten, wenn sie in Einheitsdosen vorliegen und für die Applikation z.B. am Menschen bestimmt sind, etwa 0,5 bis 1000 mg, vorteilhafterweise 1 bis 400 mg und insbesondere 5 bis 300 mg Wirkstoff.

Im allgemeinen hat es sich sowohl in der Human- als auch in der Veterinärmedizin als vorteilhaft erwiesen, den oder die Wirkstoffe bei oraler Gabe in einer Tagesdosis von etwa 0,01 bis etwa 40, vorzugsweise 0,1 bis 25, insbesondere 0,1 bis 15 mg/kg Körpergewicht, gegebenenfalls in Form mehrerer, vorzugsweise 2 bis 3 Einzelgaben zur Erzielung der gewünschten Ergebnisse zu verabreichen. Eine Einzelgabe enthält den oder die Wirkstoffe in Mengen von etwa 0,01 bis etwa 20, vorzugsweise 0,1 bis 15, insbesondere 0,1 bis 10 mg/kg Körpergewicht.

Bei einer parenteralen Behandlung, z.B. einer intramuskulären oder intravenösen Applikation, können ähnliche Dosierungen zur Anwendung kommen. Bei dieser Therapie werden etwa 50 bis 1000 mg Wirkstoff appliziert.

Die therapeutische Verabreichung der pharmazeutischen Zubereitung erfolgt bei Dauermedikation im allgemeinen zu festgelegten Zeitpunkten, wie 1 bis 4 mal am Tage, z.B. jeweils nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann der Fachmann aufgrund seines Fachwissens jederzeit vornehmen.

Die pharmazeutischen Zubereitungen bestehen in der Regel aus den erfindungsgemässen Verbindungen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzuges, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süssungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass eine verzögerte Auflösung und Resorption des Arzneimittels im Gastrointestinaltrakt und damit z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Olivenöl-, Erdnuss- oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süssungsmittel, z.B. Saccharose, Lactose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süssungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den obengenannten, sowie mit Süssungsmitteln, Geschmacksmitteln und Farbstoffen enthalten.

Emulsionen können z.B. Oliven-, Erdnuss- oder Paraffinöl neben Emulgiermitteln, wie z.B. Akaziengummi, Traganthgummi, Phosphatiden, Sorbitanmonooleat, Polyoxyethylensorbitanmonooleat, und Süssungs- und Geschmacksmitteln enthalten.

Zur rektalen Anwendung der Arzneistoffe gelangen Suppositorien, die mit Hilfe von bei Rektaltemperatur schmelzenden Bindemitteln, beispielsweise Kakaobutter oder Polyethylenglykolen, hergestellt werden.

Zur parenteralen Anwendung der Arzneistoffe dienen steril injizierbare wässrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten können.

Der oder die Wirkstoffe können gegebenenfalls mit einem oder mehreren der angegebenen Träger- oder Zusatzstoffe auch in mikroverkapselter Form formuliert werden.

Neben den erfindungsgemässen N-substituierten ω-Aminoalkanoyl-ω-aminoalkansäuren und/oder ihren Salzen können die pharmazeutischen Zubereitungen einen oder mehrere andere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergika, wie z.B. Oxyphencyclimin, Phencarbamid; Entschäumungsmittel, beispielsweise Dimethylpolysiloxan; Laxantien, beispielsweise Bisacodyl, Quellmittel; gegebenenfalls auch Fermente, Gallensäuren, Antibiotika, Vitamine, Aminosäuren, Fettsäuregemische etc. enthalten.

Ein weiterer Gegenstand der Erfindung ist ein Verfahren zur Herstellung der N-substituierten ω-Aminoalkanoyl-ω-aminoalkansäuren der allgemeinen Formel I, welches dadurch gekennzeichnet ist, dass man eine ω-Aminoalkansäure der allgemeinen Formel II

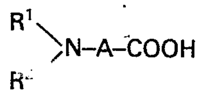

(II),

worin $R^1$, $R^2$ und A die oben angegebene Bedeutung haben, gegebenenfalls nach Überführung in die entsprechenden Säurederivate, z.B. die Säurechloride, -azide, -azolide, -anhydride oder -ester, mit einer ω-Aminoalkansäure der allgemeinen Formel III

$R^3-NH-B-COOH$ (III),

worin $R^3$ und B die oben angegebene Bedeutung haben, umsetzt.

Die Umsetzung der ω-Aminoalkansäuren der Formel II mit denen der Formel III erfolgt nach Methoden, wie sie dem Fachmann allgemein, z.B. aus der Peptidchemie, bekannt sind. Genannt seien beispielsweise folgende Verfahren: Methode der gemischten Anhydride, die Carbodiimid-Methode, die Azid-Methode, die Methode der aktivierten Ester. Gegebenenfalls werden Amino- oder Carboxylgruppen der Verbindungen II bzw. III, die nicht zur Umsetzung gelangen sollen, durch aus der Peptidchemie bekannte Schutzgruppen geschützt und nach der Reaktion abgespalten. Eine allgemeine Beschreibung dieser Methoden findet sich unter anderem in H. Beyer, Lehrbuch der organischen Chemie, S. Hirzel-Verlag, Leipzig (1968); Houben-Weyl, Methoden der organischen Chemie, 4. Auflage, Bd. XV/2, Synthese von Peptiden; Synthese von Peptiden, Teil II, herausgegeben von E. Wünsch, Georg Thieme Verlag Stuttgart (1974); Synthetic Polypeptides, Preparation, Structure and Properties, by C.H. Bamford et al, Academic Press Inc.,

New York (1956); Peptide Synthesis, by M. Bodanszky and M.A. Ondetti, Interscience Publishers, New York, London, Sydney (1966).

Zur Herstellung der Verbindungen der Ausgestaltungen I*, I** bzw. I*** werden entsprechende Ausgangsmaterialien II*, II** bzw. II*** und III*, III** bzw. III***, worin die Substituierten die oben angegebene Bedeutung haben, miteinander umgesetzt.

Die Ausgangsaminoalkansäuren der Formel II und III sind bekannt oder werden nach bekannten Verfahren hergestellt, wie zum Beispiel:

p-Anisidinoessigsäure-ethylester wird in einem inerten Lösungsmittel (Benzol) gelöst und in Gegenwart der äquivalenten Menge Pyridin mit der äquivalenten Menge p-Chlorobenzoylchlorid versetzt. Der entstandene N-p-Chlorbenzoyl-p-anisinoessigsäure-ethylester wird mit alkoholischer Kalilauge verseift. Man erhält N-p-Chlorbenzoyl-p-anisidino-essigsäure, Schmp. 195–197° C.

Analog erhält man aus 2,6-Dimethylanilinoessigsäure-ethylester durch Umsetzung mit p-Chlorbenzoylchlorid und Verseifung des Reaktionsproduktes N-p-Chlorbenzoyl-2,6-dimethylanilinoessigsäure, Schmp. 182–184° C.

Analog dem Herstellungsverfahren für N-Benzoyl-β-(p-anisidino)-propionsäure [R.C. Elderfield et al, J. Am. chem. Soc. 68 (1946) 1262–1263] werden durch Reaktion der entsprechenden Ausgangsverbindungen erhalten:

N-p-Chlorbenzoyl-β-(p-anisidino)-propionsäure, Schmp. 70–74° C;

N-Acetyl-β-(p-anisidino)-propionsäure, Öl;

N-p-Chlorbenzoyl-β-(2,6-dimethylanilino)-propionsäure, Schmp. 162–163° C.

Analog wird durch Acylierung von α-(p-Anisidino)-propionsäure-ethylester und nachfolgende Verseifung des Reaktionsproduktes erhalten:

N-Acetyl-α-(p-anisidino)-propionsäure, Schmp. 195–197° C;

N-p-Chlorbenzoyl-α-(p-anisidino)-propionsäure, Schmp. 164–166° C;

N-2,4-Dichlorbenzoyl-α-(p-anisidino)-propionsäure, Schmp. 143–145° C;

N-m-Trifluormethylbenzoyl-α-(p-anisidino)-propionsäure, Öl;

N-α-Furoyl-α-(p-anisidino)-propionsäure, Schmp. 156–158° C.

Durch Acylierung von γ-(2,6-Dimethylanilino)-buttersäure-ethylester mit 3,4,5-Trimethoxybenzoylchlorid und nachfolgende Verseifung wird erhalten:

N-(3,4,5-Trimethoxybenzoyl)-γ-(2,6-dimethylanilino)-buttersäure, Schmp. 134–136° C.

Verbindungen der Formel III, vorzugsweise Niederalkylester, werden leicht durch Umsetzung von ω-Bromalkansäureestern mit primären Aminen erhalten. Die Acylierung ergibt N-Acylaminosäureester, deren Verseifung die freien Säuren der Formel II liefert. So werden beispielsweise folgende neue Verbindungen hergestellt:

γ-(p-Anisidino)-buttersäure-ethylester, Schmp. 39–40° C;

γ-(2,6-Diethylanilino)-buttersäure-ethylester, Sdp. 124–126° C (0,05 Torr);

γ-(2-Ethyl-6-methyl-anilino)-buttersäure-ethylester, Sdp. 112–120° C (0,05 Torr);

N-p-Chlorbenzoyl-4-[(1.1.3.3-tetramethylbutyl)-amino]-buttersäure, Schmp. 141–143° C (aus 4-Brombuttersäure-ethylester und 1.1.3.3.-Tetramethylbutylamin, Acylierung des 4-[(1.1.3.3-Tetramethylbutyl)-amino]-buttersäure-ethylesters mit p-Chlorbenzoylchlorid zu N-p-Chlorbenzoyl-4-[(1.1.3.3-tetramethylbutyl)-amino]-buttersäure-ethylester, Schmp. 79–81° C und Verseifung);

N-p-Fluorbenzoyl-4-[1.1.3.3-tetramethylbutyl)-amino]-buttersäure, Schmp. 114–117° C (aus 4-Brombuttersäureethylester und 1.1.3.3-Tetramethylbutylamin, Acylierung des 4-[1.1.3.3-Tetramethylbutyl)-amino]-buttersäureethylesters mit p-Fluorbenzoylchlorid zu N-p-Fluorbenzoyl-4-[1.1.3.3-tetramethylbutyl)-amino]-buttersäure-ethylester [zähes Öl] und Verseifung);

N-p-Chlorbenzoyl-4-(tert.-butylamino)-buttersäure, Schmp. 126–127° C (aus 4-Brombuttersäure-ethylester und tert.-Butylamin, Acylierung des 4-(tert.-Butylamino)-buttersäureethylesters und Verseifung);

N-3,4,5-Trimethoxybenzoyl-6-(tert.-butylamino)-capronsäure, Schmp. 83–85° C (aus 6-Bromcapronsäureethylester und tert.-Butylamin, Acylierung des 6-(tert.-Butylamino)-capronsäure-ethylesters mit 3,4,5-Trimethoxybenzoylchlorid zu N-3,4,5-Trimethoxybenzoyl-6-(tert.-butylamino)-capronsäure-ethylester [zähes, nicht unzersetzt destillierbares Öl] und Verseifung);

N-p-Chlorbenzoyl-4-[1.1-dimethylpropyl)-amino]-buttersäure, Schmp. 79–81° C (aus 4-Brombuttersäure-ethylester und 1.1-Dimethylpropylamin, Acylierung des 4-[1.1-Dimethylpropyl)-amino]-buttersäureethylester mit p-Chlorbenzoylchlorid zu N-p-Chlorbenzoyl-4-[(1.1-dimethylpropyl)-amino]-buttersäure-ethylester [F. 65–67° C] und Verseifung);

N-2,4-Dichlorbenzoyl-4-[(1.1-dimethylpropyl)-amino]-buttersäure, Schmp. 124–126° C (aus 4-Brombuttersäure-ethylester und 1.1-Dimethylpropylamin, Acylierung des 4-[(1.1-dimethyl-propyl)-amino]-buttersäure-ethylesters mit 2,4-Dichlorbenzoylchlorid zu N-2,4-Dichlorbenzoyl-4-[(1.1-dimethylpropyl)-amino]-buttersäure-ethylester [F. 75–77° C] und Verseifung);

N-n-Butyryl-4-[1.1-dimethylpropyl)-amino]-buttersäure, Schmp. 70–72° C (aus 4-Brombuttersäure-ethylester und 1.1-Dimethylpropylamin, Acylierung des 4-[1.1-Dimethylpropyl)-amino]-buttersäure-ethylesters mit n-Buttersäure-anhydrid zu N-n-Butyryl-4-[(1.1-dimethylpropyl)-amino]-buttersäure-ethylester [Öl] und Verseifung);

N-p-Chlorbenzoyl-4-[(2-methyl-3-butin-2-yl)-amino]-buttersäure, Schmp. 102–104° C (aus 4-Brombuttersäure-ethylester und 2-Methyl-3-butin-2-yl-amin, Acylierung des 4-[(2-Methyl-3-butin-2-yl)-amino]-buttersäure-ethylesters mit p-Chlorbenzoylchlorid zu N-p-Chlorbenzoyl-4-[(2-methyl-3-butin-2-yl)-amino]-buttersäure-ethylester [F. 68–70° C] und Verseifung);

N-p-Chlorbenzoyl-4-[(3-ethyl-1-pentin-3-yl)-amino]-buttersäure, Schmp. 92–94° C (aus 4-Brombuttersäure-ethylester und 3-Ethyl-1-pentin-3-yl-amin, Acylierung des 4-[(3-Ethyl-1-pentin-3-yl)-amino]-buttersäure-ethylesters mit p-Chlorbenzoylchlorid zu N-p-Chlorbenzoyl-4-[(3-ethyl-1-pentin-3-yl)-amino]-buttersäure-ethylester [F. 73–75° C] und Verseifung);

N-p-Chlorbenzoyl-4-[(1-ethinyl-cyclohexyl-1)-amino]-buttersäure, Schmp. 120–122° C (aus 4-Brombuttersäure-ethylester und 1-Ethinyl-cyclohexylamin, Acylierung des 4-[(1-Ethinyl-cyclohexyl-1)-amino]-buttersäure-ethylester mit p-Chlorbenzoylchlorid zu N-p-Chlorbenzoyl-4-[(1-ethinyl-cyclohexyl-1)-amino]-buttersäure-ethylester [F. 84–86° C] und Verseifung);

N-Acetyl-4-[(1-ethinyl-cyclohexyl-1)-amino]-buttersäure, Schmp. 103–105° C, (aus 4-Brombuttersäure-ethylester und 1-Ethinyl-cyclohexylamin, Acylierung des 4-[(1-Ethinyl-cyclohexyl-1)-amino]-buttersäure-ethylesters mit Acetylchlorid zu N-Acetyl-4-[(ethinyl-cyclohexyl-1)-amino]-buttersäure-ethylester [F. 73–75° C] und Verseifung);

N-p-Chlorbenzoyl-4-[(propyl-cyclohexyl-1)-amino]-buttersäure, Schmp. 110–112° C (aus 4-Brombuttersäure-ethylester und 1-n-Propylcyclohexylamin, Acylierung des 4-[(1-n-Propyl-cyclohexyl-1)-amino]-buttersäure-ethylesters mit p-Chlorbenzoylchlorid zu N-p-Chlorbenzoyl-4-[(1-n-propyl-cyclohexyl-1)-amino]-buttersäure-ethylester [zähes, nicht unzersetzt destillierbares Öl] und Verseifung);

N-p-Chlorbenzoyl-4-[(1-n-butyl-cyclopentyl-1)-amino]-buttersäure, Schmp. 91–93° C (aus 4-Brombuttersäure-ethylester und 1-n-Butyl-cyclopentylamin, Acylierung des 4-[(1-n-Butyl-cyclopentyl-1)-amino]-buttersäure-ethylesters mit p-Chlorbenzoylchlorid zu N-p-Chlorbenzoyl-4-[(1-n-butyl-cyclopentyl-1)-amino]-buttersäure-ethylester [F. 85–87° C] und Verseifung);

N-p-Chlorbenzoyl-4-(1-adamantyl)-aminobuttersäure, Schmp. 164–166° C (aus 4-Brombuttersäure-ethylester und 1-Aminoadamatan, Acylierung des 4-[(1-Adamantly)-amino]-buttersäure-ethylesters mit p-Chlorbenzoylchlorid zu N-p-Chlorbenzoyl-4-[(1-adamantyl)-amino]-buttersäure-ethylester [F. 103–105° C] und Verseifung);

N-p-Chlorbenzoyl-4-cyclooctylamino-buttersäure, Schmp. 109–110° C (aus 4-Brombuttersäure-ethylester und Cyclooctylamin, Acylierung des 4-Cyclooctylamino-buttersäure-ethylesters mit p-Chlorbenzoyl-chlorid zu N-p-Chlorbenzoyl-4-cyclooctylamino-buttersäure-ethylester [nicht unzersetzt destillierbares Öl] und Verseifung);

N-p-Chlorbenzoyl-4-benzhydrylamino-buttersäure, Schmp. 110–111° C (aus 4-Brombuttersäure-ethylester und Benzhydrylamin, Acylierung des 4-Benzhydrylamino-buttersäure-ethylesters [Sdp. 150–155° C (0,02 Torr)] mit p-Chlorbenzoylchlorid zu N-p-Chlorbenzoyl-4-benzhydrylamino-buttersäure-ethylester [F. 68–69° C] und Verseifung);

N-Acetyl-4-benzhydrylamino-buttersäure, Schmp. 173–174° C (durch Acylierung von 4-Benzhydrylamino-buttersäure-ethylester mit Acetylchlorid zu N-Acetyl-4-benzhydrylamino-buttersäure-ethylester [zähes, nicht destillierbares Öl] und Verseifung);

N-p-Chlorbenzoyl-4-(1-phenylethyl-amino)-buttersäure, Schmp. 110–112° C (aus 4-Brombuttersäure-ethylester und dl-1-phenylethylamin, Acylierung des 4-(1-Phenylethylamino)-buttersäure-ethylesters mit p-Chlorbenzoylchlorid zu N-p-Chlorbenzoyl-4-(1-phenylethylamino)-buttersäure-ethylester [zähes, nicht destillierbares Öl] und Verseifung);

N-p-Chlorbenzoyl-6-(1-phenylethylamino)-capronsäure, Schmp. 132–133° C (aus 6-Bromcapronsäure-ethylester und dl-1-Phenylethylamin, Acylierung des 6-(1-Phenylethylamino)-capronsäure-ethylesters mit p-Chlorbenzoylchlorid zu N-p-Chlorbenzoyl-6-(1-phenylethylamino)-capronsäure-ethylester [zähes, nicht destillierbares Öl] und Verseifung);

N-p-Chlorbenzoyl-4-homoveratrylamino-buttersäure, Schmp. 101–103° C (aus 4-Brombuttersäure-ethylester und Homoveratrylamin, Acylierung des 4-Homoveratrylamino-buttersäureethylesters mit p-Chlorbenzoylchlorid zu N-p-Chlorbenzoyl-4-homoveratrylamino-buttersäure-ethylester [zähes, nicht destillierbares Öl] und Verseifung);

N-p-Chlorbenzoyl-4-[(1,2-diphenylethyl)-amino]-buttersäure, Schmp. 121–122° C (aus 4-Brombuttersäure-ethylester und 1,2-Diphenylethylamin, Acylierung des 4-[(1,2-Diphenylethyl)-amino]-buttersäure-ethylesters mit p-Chlorbenzoylchlorid zu N-p-Chlorbenzoyl-4-[1,2-diphenylethyl)-amino]-buttersäure-ethylester [zähes, nicht destillierbares Öl] und Verseifung);

N-p-Chlorbenzoyl-4-aminobuttersäure, Schmp. 107–108° C (durch Acylierung von 4-Aminobuttersäure mit p-Chlorbenzylchlorid in Natriumhydroxidlösung bei pH- 7–8);

N-m-Trifluormethyl-benzoyl-4-[(1.1.3.3-tetramethylbutyl)-amino]-buttersäure, Schmp. 86–87° C (aus 4-Brombuttersäure-ethylester und 1.1.3.3-Tetramethylbutylamin, Acylierung des 4-[(1.1.3.3-Tetramethylbutyl)-amino]-buttersäure-ethylesters mit m-Trifluor-methylbenzoylchlorid und Verseifung);

N-Crotonoyl-4-[(1.1.3.3-tetramethylbutyl)-amino]-buttersäure, Schmp. 92–93° C (aus 4-Brombuttersäure-ethylester und 1.1.3.3-Tetramethylbutylamin, Acylierung des 4-[(1.1.3.3-Tetramethylbutyl)-amino]-buttersäure-ethylesters mit Crotonsäurechlorid zu N-Crotonoyl-4-[1.1.3.3-tetramethylbutyl)-amino]-buttersäure-ethylester [zähes, Öl] und Verseifung);

N-Propionyl-4-benzhydrylamino-buttersäure, Schmp. 151,5–152,5° C (durch Acylierung von 4-Benzhydrylamino-buttersäure-ethylester mit Propionylchlorid zu N-Propionyl-4-benzhydrylamino-buttersäure-ethylester [F.83–85° C] und Verseifung);

N-(5-Chlor-2-methoxy-benzoyl)-4-benzhydrylamino-buttersäure, Schmp. 176–178° C (durch Acylierung von 4-Benzhydrylamino-buttersäure-ethylester mit 5-Chlor-2-methoxy-benzoesäurechlorid zu N-(5-Chlor-2-methoxy-benzoyl)-4-benzhydrylamino-buttersäure-ethylester [zähes, nicht destillierbares Öl] und Verseifung);

N-Acetyl-6-benzhydrylamino-capronsäure, Schmp. 119–120° C (aus 6-Bromcapronsäure-

ethylester und Benzhydrylamin, Acylierung des 6-Benzhydrylamino-capronsäure-ethylesters [Sdp. 162–167° C (0,02 Torr)] mit Acetylchlorid zu N-Acetyl-6-benzhydrylamino-capronsäure-ethylester [zähes, nicht destillierbares Öl] und Verseifung);

N-Isobutyryl-6-benzhydrylamino-capronsäure, Schmp. 106–107° C (durch Acylierung von 6-Benzhydrylamino-capronsäure-ethylester mit Isobutyrylchlorid zu N-Isobutyryl-6-benzhydrylamino-capronsäure-ethylester [zähes, nicht destillierbares Öl] und Verseifung);

N-Acetyl-5-benzhydrylamino-valeriansäure, Schmp. 135–136° C (aus 5-Bromvaleriansäureethylester und Benzhydrylamin, Acylierung des 5-Benzhydrylamino-valeriansäure-ethylesters [Sdp. 158–163° C (0,01 Torr)] mit Acetylchlorid zu N-Acetyl-5-benzhydrylamino-valeriansäure-ethylester [zähes, nicht destillierbares Öl] und Verseifung);

N-Crotonoyl-5-benzhydrylamino-valeriansäure, Schmp. 88–89° C (durch Acylierung von 5-Benzhydrylamino-valeriansäure-ethylester mit Crotonoylchlorid zu N-Crotonoyl-5-benzhydrylamino-valeriansäure-ethylester [zähes, nicht destillierbares Öl] und Verseifung).

Die Vorprodukte II sind auch durch Hydrolyse (Verseifung) der entsprechenden N-$R^2$-Lactame und anschliessende Acylierung herstellbar. Als Beispiele der nach dieser Variante hergestellten Ausgangsprodukte seien genannt:

N-Benzoyl-4-n-butylamino-buttersäure, Schmp. 62–64° C (durch Verseifung von n-Butylpyrrolidon mit Natriumhydroxid und anschliessende Acylierung mit Benzoylchlorid);

N-p-Chlorbenzoyl-5-(n-butylamino)-valeriansäure, Schmp. 64,5–65,5° C (durch Verseifung von 1-n-Butyl-δ-valerolactam mit Natriumhydroxid und anschliessende Acylierung mit p-Chlorbenzoylchlorid bei pH 7 bis 8; 1-n-Butyl-δ-valerolactam [Sdp. 122° C/13 Torr] wird durch Alkylierung von δ-Valerolactam mit 1-Brombutan in wasserfreiem Dimethylsulfoxid in Gegenwart von Kaliumhydroxid erhalten);

N-p-Chlorbenzoyl-4-benzylamino-buttersäure, Schmp. 101–102° C (durch Verseifung von N-Benzylpyrrolidon mit Natriumhydroxid und anschliessende Acylierung mit p-Chlorbenzoylchlorid);

N-p-Chlorbenzoyl-4-[(p-methoxybenzyl)-amino]-buttersäure, Schmp. 128,5–129,5° C (durch Verseifung von 1-p-Methoxy-benzylpyrrolidon mit Natriumhydroxid und anschliessende Acylierung mit p-Chlorbenzoylchlorid);

N-p-Chlorbenzoyl-5-benzylamino-valeriansäure, Schmp. 93–94° C (durch Verseifung von 1-Benzyl-δ-valerolactam mit Natriumhydroxid und anschliessende Acylierung mit p-Chlorbenzoylchlorid).

Die Überführung der Säuren der allgemeinen Formel I oder der Ausgestaltungen I*, I** und I*** in ihre Salze kann durch direkte alkalische Hydrolyse der Derivate, z.B. der Ester, von Säuren der allgemeinen Formel I (bzw. I*, I** und I***) erfolgen. Als alkalischer Reaktionspartner wird diejenige anorganische oder organische Base verwendet, deren Salz gewünscht wird. Man erhält die Salze aber auch, indem man die Säuren der allgemeinen Formel I (bzw. I*, I** und I***) mit dem stöchiometrischen Äquivalent an entsprechender Base, z.B. Natriumhydroxid oder Natriumalkoholat, umsetzt, oder leicht lösliche Salze durch doppelte Umsetzung in schwer lösliche Salze überführt, oder beliebige Salze in pharmakologisch verträgliche Salze überführt.

Die folgenden Beispiele erläutern die Erfindung näher, ohne sie einzuschränken. Die Abkürzungen Schmp. bzw. Sdp. bedeuten Schmelzpunkt bzw. Siedepunkt, die Temperaturangaben erfolgen in ° C.

**Beispiele**

Beispiel 1
N-[N-p-Chlorbenzoyl-4-(2,6-dimethylanilino)-butyryl]-4-(p-anisidino)-buttersäure
$R^1$ = p-Chlorbenzoyl, $R^2$ = 2,6-Dimethylphenyl, $R^3$ = p-Methoxyphenyl,
A = B = $-CH_2-CH_2-CH_2-$

a) 34,6 g N-p-Chlorbenzoyl-4-(2,6-dimethylanilino)-buttersäure werden in 100 ml frisch destilliertem Tetrahydrofuran gelöst und mit 10,1 g Triethylamin versetzt. Die Lösung wird in einem Kältebad auf −15° gekühlt; nach Zutropfen von 5,4 g Chlorameisensäureethylester wird noch weitere 10 Minuten bei −15° gerührt. Bei dieser Temperatur wird dann anschliessend eine Lösung von 22,3 g 4-(p-Anisidino)-buttersäure-ethylester in 60 ml Tetrahydrofuran zugegeben. Das Kältebad wird danach entfernt, es wird weitere 20 Stunden bis zur Beendigung der Kohlendioxid-Entwicklung gerührt. Das Tetrahydrofuran wird im Vakuum abdestilliert und der Rückstand in Essigsäureethylester aufgenommen. Die Essigesterlösung wird, in der angegebenen Reihenfolge, je dreimal mit 1n Salzsäure, Wasser, 5%iger Kaliumhydrogencarbonat-Lösung und einmal mit Wasser ausgeschüttelt. Die organische Phase wird über Magnesiumsulfat getrocknet und das Lösungsmittel abgedampft. Der Eindampfrückstand wird aus Essigsäureethylester-Cyclohexan umkristallisiert. Es werden 44,0 g (75% d.Th.) N-[N-p-Chlorbenzoyl-4-(2,6-dimethylanilino)-butyryl]-4-(p-anisidino)-buttersäure-ethylester (Schmp. 99–101°) erhalten.

b) Eine Lösung von 33,9 g des nach a) erhaltenen Esters in 200 ml Benzol und eine Lösung von 3,9 g Kaliumhydroxid in 40 ml Ethanol werden vermischt und anschliessend 12 Stunden bei Raumtemperatur gerührt. Die Lösung wird zweimal mit je 150 ml Wasser ausgeschüttelt; die vereinigten wässrigen Phasen werden mit Diethylether gewaschen und dann mit verdünnter Salzsäure angesäuert. Der entstandene Niederschlag wird mit Chloroform extrahiert. Der nach dem Abdestillieren des Chloroform verbleibende Rückstand wird aus Essigsäureethylester umkristallisiert. Es werden 28,7 g (89% d.Th.) der Titelverbindung (Schmp. 119–121°) erhalten.

Beispiel 2

N-[N-(p-Chlorbenzoyl)-4-(2,6-dimethylanilino)-butyryl]-4-(2,6-dimethylanilino)-buttersäure

$R^1$ = p-Chlorbenzoyl, $R^2$ = $R^3$ = 2,6-Dimethylphenyl,

A = B =$-CH_2-CH_2-CH_2-$

Analog Beispiel 1 wird bei Einsatz äquivalenter Mengen durch Umsetzung von N-(p-Chlorbenzoyl)-4-(2,6-dimethylanilino)-buttersäure und 4-(2,6-Dimethylanilino)-buttersäure-ethylester und entsprechender Aufarbeitung N-[N-(p-Chlorbenzoyl)-4-(2,6-dimethylanilino)-butyryl]-4-(2,6-dimethylanilino)-buttersäure-ethylester (Schmp. 95–97°) erhalten, dessen Verseifung und Aufarbeitung des Reaktionsproduktes die Titelverbindung (Schmp. 129–131°) ergibt.

Beispiel 3

N-[N-(p-Chlorbenzoyl)-4-(p-anisidino)-butyryl]-4-(2,6-dimethylanilino)-buttersäure

$R^1$ = p-Chlorbenzoyl, $R^2$ = p-Methoxyphenyl, $R^3$ = 2,6-Dimethylphenyl,

A = B = $-CH_2-CH_2-CH_2-$

Analog Beispiel 1 wird bei Einsatz äquivalenter Mengen durch Umsetzung von N-(p-Chlorbenzoyl)-4-(p-anisidino)-buttersäure und 4-(2,6-Dimethylanilino)-buttersäure-ethylester und entsprechender Aufarbeitung N-[N-(p-Chlorbenzoyl)-4-(p-anisidino)-butyryl]-4-(2,6-diemthylanilino)-buttersäure-ethylester (Öl) erhalten, dessen Verseifung und Aufarbeitung des Reaktionsproduktes die Titelverbindung (Schmp. 131–133°) ergibt.

Beispiel 4

N-[N-p-Chlorbenzoyl-4-(p-anisidino)-butyryl]-4-(p-anisidino)-buttersäure

$R^1$ = p-Chlorbenzoyl, $R^2$ = $R^3$ = p-Methoxyphenyl,

A = B = $-CH_2-CH_2-CH_2-$

Analog Beispiel 1 wird bei Einsatz äquivalenter Mengen durch Umsetzung von N-p-Chlorbenzoyl-4-(p-anisidino)-buttersäure und N-p-Chlorbenzoyl-4-(p-anisidino)-buttersäuremethylester und entsprechender Aufarbeitung N-[N-p-Chlorbenzoyl-4-(p-anisidino)-butyryl]-4-(p-anisidino)-buttersäure-methylester (Öl) erhalten, dessen Verseifung und Aufarbeitung des Reaktionsproduktes die Titelverbindung (Schmp. 62–64°) ergibt.

Beispiel 5

N-[N-p-Chlorbenzoyl-4-(2,6-dimethylanilino-butyryl]-4-aminobuttersäure

$R^1$ = p-Chlorbenzoyl, $R^2$ = 2,6-Dimethylphenyl, $R^3$ = -H, A = B = $-CH_2-CH_2-CH_2-$

Analog Beispiel 1 wird bei Einsatz äquivalenter Mengen durch Umsetzung von N-p-Chlorbenzoyl-4-(2,6-dimethylanilino)-buttersäure und 4-Aminobuttersäure-methylester und entsprechender Aufarbeitung N-[N-p-Chlorbenzoyl-4-(2,6-dimethylanilino)-butyryl]-4-aminobuttersäure-methylester (Öl) erhalten, dessen Verseifung und Aufarbeitung des Reaktionsproduktes die Titelverbindung (Schmp. 141–142°) ergibt.

Beispiel 6

N-[N-(3,4,5-Trimethoxybenzoyl)-4-(2,6-dimethylanilino)-butyryl]-4-amino-buttersäure

$R^1$ = 3,4,5-Trimethoxybenzoyl, $R^2$ = 2,6-Dimethylphenyl, $R^3$ = -H, A = B = $-CH_2-CH_2-CH_2-$

Analog Beispiel 1 wird bei Einsatz äquivalenter Mengen durch Umsetzung von N-3,4,5-Trimethoxybenzoyl-4-(2,6-dimethylanilino)-buttersäure und 4-Aminobuttersäure-methylester und entsprechender Aufarbeitung N-[N-(3,4,5-Trimethoxybenzoyl)-4-(2,6-dimethylanilino)-butyryl]-4-aminobuttersäure-methylester (Schmp. 97–99°) erhalten, dessen Verseifung und Aufarbeitung des Reaktionsproduktes die Titelverbindung (Schmp. 100–101°) ergibt.

Beispiel 7

N-[N-(p-Chlorbenzoyl)-3-(p-anisidino)-propionyl]-4-(p-anisidino)-buttersäure

$R^1$ = p-Chlorbenzoyl, $R^2$ = $R^3$ = p-Methoxyphenyl,

A = $-CH_2-CH_2-$, B = $-CH_2-CH_2-CH_2-$

Analog Beispiel 1 wird bei Einsatz äquivalenter Mengen durch Umsetzung von N-(p-Chlorbenzoyl)-3-(p-anisidino)-propionsäure und 4-(p-Anisidino)-buttersäure-methylester und entsprechender Aufarbeitung N-[N-(p-Chlorbenzoyl)-3-(p-anisidino)-propionyl]-4-(p-anisidino)-buttersäure-methylester (Öl) erhalten, dessen Verseifung und Aufarbeitung des Reaktionsproduktes die Titelverbindung (Schmp. 91–93°) ergibt.

Beispiel 8

N-[N-(p-Chlorbenzoyl)-3-(2,6-dimethylanilino)-propionyl]-4-(p-anisidino)-buttersäure

$R^1$ = p-Chlorbenzoyl, $R^2$ = 2,6-Dimethylphenyl, $R^3$ = p-Methoxyphenyl,

A = $-CH_2-CH_2-$, B = $-CH_2-CH_2-CH_2-$

Analog Beispiel 1 wird bei Einsatz äquivalenter Mengen durch Umsetzung von N-(p-Chlorbenzoyl)-3-(2,6-dimethylanilino)-propionsäure und 4-(p-Anisidino)-buttersäure-methylester und entsprechender Aufarbeitung N-[N-p-Chlorbenzoyl)-3-(2,6-dimethylanilino)-propionyl]-4-(p-anisidino)-buttersäure-methylester (Öl) erhalten, dessen Verseifung und Aufarbeitung des Reaktionsproduktes die Titelverbindung (Schmp. 138–140°) ergibt.

Beispiel 9

N-[N-Acetyl-3-(2,6-dimethylanilino)-propionyl]-4-(p-anisidino)-buttersäure

$R^1$ = $CH_3$-CO-, $R^2$ = 2,6-Dimethylphenyl, $R^3$ = p-Methoxyphenyl,

A = $-CH_2-CH_2-$, B = $-CH_2-CH_2-CH_2-$

Analog Beispiel 1 wird bei Einsatz äquivalenter Mengen durch Umsetzung von N-Acetyl-3-(2,6-dimethylanilino)-propionsäure und 4-(p-Anisidino)-buttersäure-methylester und entsprechender Aufarbeitung N-[N-Acetyl-3-(2,6-dimethylanilino)-propionyl]-4-(p-anisidino)-buttersäure-methylester (Öl) erhalten, dessen Verseifung und Aufarbeitung des Reaktionsproduktes die Titelverbindung (Schmp. 90–92°) ergibt.

Beispiel 10

N-[N-Acetyl-3-(2,6-dimethylanilino)-propionyl]-4-(2,6-diethylanilino)-buttersäure

$R^1$ = $CH_3$-CO-, $R^2$ = 2,6-Dimethylphenyl, $R^3$ = 2,6-Diethylphenyl,
A = -$CH_2$-$Ch_2$, B = -$CH_2$-$CH_2$-$CH_2$-

Analog Beispiel 1 wird bei Einsatz äquivalenter Mengen durch Umsetzung von N-Acetyl-3-(2,6-dimethylanilino)-propionsäure und 4-(2,6-Diethylanilino)-buttersäure-ethylester und entsprechender Aufarbeitung N-[N-Acetyl-3-(2,6-dimethylanilino)-propionyl]-4-(2,6-diethylanilino)-buttersäure-ethylester (Öl) erhalten, dessen Verseifung und Aufarbeitung des Reaktionsproduktes die Titelverbindung (Schmp. 110–111°) ergibt.

Beispiel 11
N-[N-Acetyl-3-(2,6-dimethylanilino)-propionyl]-4-(2-ethyl-6-methylanilino)-buttersäure
$R^1$ = $CH_3$-CO-, $R^2$ = 2,6-Dimethylphenyl, $R^3$ = 2-Ethyl-6-methylphenyl,
A = -$CH_2$-$CH_2$-, B = -$CH_2$-$CH_2$-$CH_2$-

Analog Beispiel 1 wird bei Einsatz äquivalenter Mengen durch Umsetzung von N-Acetyl-3-(2,6-dimethylanilino)-propionsäure und 4-(2-Ethyl-6-methylanilino)-buttersäure-ethylester und entsprechender Aufarbeitung N-[N-Acetyl-3-(2,6-dimethylanilino)-propionyl]-4-(2-ethyl-6-methyl-anilino)-buttersäure-ethylester (Öl) erhalten, dessen Verseifung und Aufarbeitung des Reaktionsproduktes die Titelverbindung (Schmp. 139–141°) ergibt.

Beispiel 12
N-[N-(p-Chlorbenzoyl)-2-(2,6-dimethylanilino)-acetyl]-4-(2,6-dimethylanilino)-buttersäure
$R^1$ = p-Chlorbenzoyl, $R^2$ = $R^3$ = 2,6-Dimethylphenyl,
A = -$CH_2$-, B = -$CH_2$-$CH_2$-$CH_2$-

Analog Beispiel 1 wird bei Einsatz äquivalenter Mengen durch Umsetzung von N-(p-Chlorbenzoyl)-2-(2,6-dimethylanilino)-essigsäure und 4-(2,6-Dimethylanilino)-buttersäure-ethylester und entsprechender Aufarbeitung N-[N-(p-Chlorbenzoyl)-2-(2,6-dimethylanilino)-acetyl]-4-(2,6-dimethylanilino)-buttersäure-ethylester (Schmp. 150–151°) erhalten, dessen Verseifung und Aufarbeitung des Reaktionsproduktes die Titelverbindung (Schmp. 214–216°) ergibt.

Beispiel 13
N-[N-(p-Chlorbenzoyl)-2-(p-anisidino)-acetyl]-4-(p-anisidino)-buttersäure
$R^1$ = p-Chlorbenzoyl, $R^2$ = $R^3$ = p-Methoxyphenyl,
A = -$CH_2$-, B = -$CH_2$-$CH_2$-$CH_2$-

Analog Beispiel 1 wird bei Einsatz äquivalenter Mengen durch Umsetzung von N-(p-Chlorbenzoyl)-2-(p-anisidino)-essigsäure und 4-(p-Anisidino)-buttersäure-methylester und entsprechender Aufarbeitung N-[N-(p-Chlorbenzoyl)-2-(p-anisidino)-acetyl]-4-(p-anisidino)-buttersäure-methylester (Öl) erhalten, dessen Verseifung und Aufarbeitung des Reaktionsproduktes die Titelverbindung (Schmp. 105–108°) ergibt.

Beispiel 14
N-[N-Acetyl-2-(p-anisidino)-acetyl]-4-(p-anisidino)-buttersäure
$R^1$ = $CH_3$-CO-, $R^2$ = $R^3$ = p-Methoxyphenyl,
A = -$CH_2$-, B = -$CH_2$-$CH_2$-$CH_2$-

Analog Beispiel 1 wird bei Einsatz äquivalenter Mengen durch Umsetzung von N-Acetyl-2-(p-anisidino)-essigsäure und 4-(p-Anisidino)-buttersäure-methylester und entsprechender Aufarbeitung N-[N-Acetyl-2-(p-anisidino)-acetyl]-4-(p-anisidino)-buttersäure-methylester (Öl) erhalten, dessen Verseifung und Aufarbeitung des Reaktionsproduktes die Titelverbindung (Schmp. 97–100°) ergibt.

Beispiel 15
N-[N-(p-Chlorbenzoyl)-2-(p-anisidino)-propionyl]-4-(p-anisidino)-buttersäure
$R^1$ = p-Chlorbenzoyl, $R^2$ = $R^3$ = p-Methoxyphenyl,
A = -CH($R^4$), $R^4$ = -$CH_3$, B = -$CH_2$-$CH_2$-$CH_2$-

Analog Beispiel 1 wird bei Einsatz äquivalenter Mengen durch Umsetzung von N-(p-Chlorbenzoyl)-2-(p-anisidino)-propionsäure und 4-(p-Anisidino)-buttersäure-methylester und entsprechender Aufarbeitung N-[N-(p-Chlorbenzoyl)-2-(p-anisidino)-propionyl]-4-(p-anisidino)-buttersäure-methylester (Öl) erhalten, dessen Verseifung und Aufarbeitung des Reaktionsproduktes die Titelverbindung (Schmp. 135–137°) ergibt.

Beispiel 16
N-[N-(2,4-Dichlorbenzoyl)-2-(p-anisidino)-propionyl]-4-(p-anisidino)-buttersäure
$R^1$ = 2,4-Dichlorbenzoyl, $R^2$ = $R^3$ = p-Methoxyphenyl, A = -CH($R^4$)-, $R^4$ = -$CH_3$, B = -$CH_2$-$CH_2$-$CH_2$-

Analog Beispiel 1 wird bei Einsatz äquivalenter Mengen durch Umsetzung von N-(2,4-Dichlorbenzoyl)-2-(p-anisidino)-propionsäure und 4-(p-Anisidino)-buttersäure-methylester und entsprechender Aufarbeitung N-[N-(2,4-Dichlorbenzoyl)-2-(p-anisidino)-propionyl]-4-(p-anisidino)-buttersäure-methylester (Öl) erhalten, dessen Verseifung und Aufarbeitung des Reaktionsproduktes die Titelverbindung (Schmp. 159–160°) ergibt.

Beispiel 17
N-[N-Acetyl-2-(p-anisidino)-propionyl]-4-(p-anisidino)-buttersäure
$R^1$ = $CH_3$-CO-, $R^2$ = $R^3$ = p-Methoxyphenyl,
A = -CH($R^4$)-, $R^4$ = -$CH_3$, B = -$CH_2$-$CH_2$-$CH_2$-

Analog Beispiel 1 wird bei Einsatz äquivalenter Mengen durch Umsetzung von N-Acetyl-2-(p-anisidino)-propionsäure und 4-(p-Anisidino)-buttersäure-methylester und entsprechender Aufarbeitung N-[N-Acetyl-2-(p-anisidino)-propionyl]-4-(p-anisidino)-buttersäure-methylester (Schmp. 111–113°) erhalten, dessen Verseifung und Aufarbeitung des Reaktionsproduktes die Titelverbindung (Schmp. 155–157°) ergibt.

Beispiel 18
N-[N-(m-Trifluormethylbenzoyl)-2-(p-anisidino)-propionyl]-4-(p-anisidino)-buttersäure
$R^1$ = m-Trifluormethylbenzoyl, $R^2$ = $R^3$ = p-Methoxyphenyl, A = -CH($R^4$)-, $R^4$ = -$CH_3$,
B = -$CH_2$-$CH_2$-$CH_2$-

Analog Beispiel 1 wird bei Einsatz äquivalenter Mengen durch Umsetzung von N-(m-Trifluorben-zoyl)-2-(p-anisidino)-propionsäure und 4-(p-Anisidino)-buttersäure-methylester und entsprechender Aufarbeitung N-[N-(m-Trifluormethylbenzoyl)-2-(p-anisidino)-propionyl]-4-(p-anisidino)-buttersäure-methylester (Öl) erhalten, dessen Verseifung und Aufarbeitung des Reaktionsproduktes die Titelverbindung (Schmp. 119–121°) ergibt.

Beispiel 19
N-[N-(2-Furoyl)-2-(p-anisidino)-propionyl]-4-(p-anisidino)-buttersäure
$R^1$ = 2-Furoyl, $R^2$ = $R^3$ = p-Methoxyphenyl,
A = $-CH(R^4)-$, $R^4$ = $-CH_3$, B = $-CH_2-CH_2-CH_2-$

Analog Beispiel 1 wird bei Einsatz äquivalenter Mengen durch Umsetzung von N-(2-Furoyl)-2-(p-anisidino)-propionsäure und 4-(p-Anisidino)-buttersäure-methylester und entsprechender Aufarbeitung N-[N-(2-Furoyl)-2-(p-anisidino)-propionyl]-4-(p-anisidino)-buttersäure-methylester (Öl) erhalten, dessen Verseifung und Aufarbeitung des Reaktionsproduktes die Titelverbindung (Schmp. 204–206°) ergibt.

Beispiel 20
N-[N-(p-Methoxybenzoyl)-L-phenylalanyl]-4-(p-anisidino)-buttersäure
$R^1$ = p-Methoxybenzoyl, $R^2$ = -H, $R^3$ = p-Methoxyphenyl, A = $-CH(R^4)-$, $R^4$ = Benzyl,
B = $-CH_2-CH_2-$ $CH_2$

Analog Beispiel 1 wird bei Einsatz äquivalenter Mengen durch Umsetzung von N-(p-Methoxybenzoyl)-L-phenylalanin und 4-(p-Anisidino)-buttersäure-methylester und entsprechender Aufarbeitung N-[N-(p-Methoxybenzoyl)-L-phenylalanyl]-4-(p-anisidino)-buttersäure-methylester (Öl) erhalten, dessen Verseifung und Aufarbeitung des Reaktionsproduktes die Titelverbindung (Schmp. 143–145°) ergibt.

Beispiel 21
N-[N-(p-Chlorbenzoyl)-phenylalanyl]-4-(p-anisidino)-buttersäure
$R^1$ = p-Chlorbenzoyl, $R^2$ = -H, $R^3$ = p-Methoxyphenyl, A = $-CH(R^4)-$, $R^4$ = Benzyl,
B = $-CH_2-CH_2-CH_2-$

Analog Beispiel 1 wird bei Einsatz äquivalenter Mengen durch Umsetzung von N-(p-Chlorbenzoyl)-phenylalanin und 4-(p-Anisidino)-buttersäure-methylester und entsprechender Aufarbeitung N-[N-(p-Chlorbenzoyl)-phenylalanyl]-4-(p-anisidino)-buttersäure-methylester (Öl) erhalten, dessen Verseifung und Aufarbeitung des Reaktionsproduktes die Titelverbindung (Schmp. 163–165°) ergibt.

Beispiel 22
N-[N-(p-Chlorbenzoyl)-L-methionyl]-4-(p-anisidino)-buttersäure
$R^1$ = p-Chlorbenzoyl, $R^2$ = -H, $R^3$ = p-Methoxyphenyl, A = $-CH(R^4)-$, $R^4$ = $CH_3-S-CH_2-CH_2-$,
B = $-CH_2-CH_2-CH_2-$

Analog Beispiel 1 wird bei Einsatz äquivalenter Mengen durch Umsetzung von N-(p-Chlorbenzoyl)-L-methionin und 4-(p-Anisidino)-buttersäure-methylester und entsprechender Aufarbeitung N-[N-(p-Chlorbenzoyl)-L-methionyl]-4-(p-anisidino)-buttersäure-methylester (Öl) erhalten, dessen Verseifung und Aufarbeitung des Reaktionsproduktes die Titelverbindung (Schmp. 150–152°) ergibt.

Beispiel 23
N-[N-(p-Chlorbenzoyl)-3-(p-anisidino)-propionyl]-3-aminopropionsäure
$R^1$ = p-Chlorbenzoyl, $R^2$ = p-Methoxyphenyl,
$R^3$ = -H, A = B = $-CH_2-CH_2-$

Analog Beispiel 1 wird bei Einsatz äquivalenter Mengen durch Umsetzung von N-(p-Chlorbenzoyl)-3-(p-anisidino)-propionsäure und 3-Aminopropionsäureethylester und entsprechender Aufarbeitung N-[N-(p-Chlorbenzoyl)-3-(p-anisidiono)-propionyl]-3-aminopropionsäureethylester (Öl) erhalten, dessen Verseifung und Aufarbeitung des Reaktionsproduktes die Titelverbindung (Schmp. 171–172°) ergibt.

Beispiel 24
N-[N-(p-Chlorbenzoyl)-4-(2,6-dimethylanilino)-butyryl]-L-alanin
$R^1$ = p-Chlorbenzoyl, $R^2$ = 2,6-Dimethylphenyl,
$R^3$ = -H, A = $-CH_2-CH_2-CH_2$, B = $-CH(R^5)-$,
$R^5$ = $-CH_3$

Analog Beispiel 1 wird bei Einsatz äquivalenter Mengen durch Umsetzung von N-(p-Chlorbenzoyl)-4-(2,6-dimethylanilino)-buttersäure und L-Alanin-methylester und entsprechender Aufarbeitung N-[N-(p-Chlorbenzoyl)-4-(2,6-dimethylanilino)-butyryl]-L-alaninmethylester (Schmp. 91–92°), erhalten, dessen Verseifung und Aufarbeitung des Reaktionsproduktes die Titelverbindung (Schmp. 159–161°) ergibt.

Beispiel 25
N-[N-(p-Chlorbenzoyl)-4-(2,6-dimethylanilino)-butyryl]-glycin
$R^1$ = p-Chlorbenzoyl, $R^2$ = 2,6-Dimethylphenyl,
$R^3$ = -H, A = $-CH_2-CH_2-CH_2-$, B = $-CH_2-$

Analog Beispiel 1 wird bei Einsatz äquivalenter Mengen durch Umsetzung von N-(p-Chlorbenzoyl)-4-(2,6-dimethylanilino)-buttersäure und Glycinethylester und entsprechender Aufarbeitung N-[N-(p-Chlorbenzoyl)-4-(2,6-dimethylanilino)-butyryl]-glycinethylester (Öl) erhalten, dessen Verseifung und Aufarbeitung des Reaktionsproduktes die Titelverbindung (Schmp. 141–142°) ergibt.

Beispiel 26
N-[N-(p-Chlorbenzoyl)-4-(2,6-dimethylanilino)-butyryl]-sarkosin
$R^1$ = p-Chlorbenzoyl, $R^2$ = 2,6-Dimethylphenyl,
$R^3$ = $-CH_3$, A = $-CH_2-CH_2-CH_2-$, B = $-CH_2-$

Analog Beispiel 1 wird bei Einsatz äquivalenter Mengen durch Umsetzung von N-(p-Chlorbenzoyl)-4-(2,6-dimethylanilino)-buttersäure und Sarkosin-ethylester und entsprechender Aufarbeitung N-[N-(p-Chlorbenzoyl)-4-(2,6-dimethylanilino)-butyryl]-sarkosin-ethylester (Öl) erhalten,

dessen Verseifung und Aufarbeitung des Reaktionsproduktes die Titelverbindung (Schmp. 103–105°) ergibt.

Beispiel 27
N-[N-(p-Chlorbenzoyl)-4-(p-anisidino)-butyryl]-glycin
$R^1$ = p-Chlorbenzyl, $R^2$ = p-Methoxyphenyl,
$R^3$ = -H, A = $-CH_2-CH_2-CH_2-$, B = $-CH_2-$

Analog Beispiel 1 wird bei Einsatz äquivalenter Mengen durch Umsetzung von N-(p-Chlorbenzoyl)-4-(p-anisidino)-buttersäure und Glycinethylester und entsprechender Aufarbeitung N-[N-(p-Chlorbenzoyl)-4-(p-anisidino)-butyryl]-glycinethylester (Öl) erhalten, dessen Verseifung und Aufarbeitung des Reaktionsproduktes die Titelverbindung (zähes Öl) ergibt.

Beispiel 28
N-[N-(p-Chlorbenzoyl)-4,(2,6-dimethylanilino)-butyryl]-L-phenylalanin
$R^1$ = p-Chlorbenzoyl, $R^2$ = 2,6-Dimethylphenyl,
$R^3$ = -H, A = $-CH_2-CH_2-CH_2$, B = $-CH(R^5)-$,
$R^5$ = Benzyl

Analog Beispiel 1 wird bei Einsatz äquivalenter Mengen durch Umsetzung von N-(p-Chlorbenzoyl)-4-(2,6-dimethylanilino)-buttersäure und L-Phenylalaninethylester und entsprechender Aufarbeitung N-[N-(p-Chlorbenzoyl)-4-(2,6-dimethylanilino)-butyryl]-L-phenylalanin-ethylester (Öl) erhalten, dessen Verseifung und Aufarbeitung des Reaktionsproduktes die Titelverbindung (Schmp. 153–155°) ergibt.

Beispiel 29
N-[N-(p-Chlorbenzoyl)-4-(2,6-dimethylanilino)-butyryl]-L-serin
$R^1$ = p-Chlorbenzoyl, $R^2$ = 2,6-Dimethylphenyl,
$R^3$ = -H, A = $-CH_2-CH_2-CH_2-$, B = $-CH(R^5)-$,
$R^5$ = $-CH_2-OH$

Analog Beispiel 1 wird bei Einsatz äquivalenter Mengen durch Umsetzung von N-(p-Chlorbenzoyl)-4-(2,6-dimethylanilino)-buttersäure und L-Serin-ethylester und entsprechender Aufarbeitung N-[N-(p-Chlorbenzoyl)-4-(2,6-dimethylanilino)-butyryl]-L-serinethylester (Öl) erhalten, dessen Verseifung und Aufarbeitung des Reaktionsproduktes die Titelverbindung (Schmp. 147–148°) ergibt.

Beispiel 30
N-[N-(p-Chlorbenzoyl)-3-(p-anisidino)-propionyl]-sarkosin
$R^1$ = p-Chlorbenzoyl, $R^2$ = p-Methoxyphenyl,
$R^3$ = $-CH_3$, A = $-CH_2-CH_2-$, B = $-CH_2-$

Analog Beispiel 1 wird bei Einsatz äquivalenter Mengen durch Umsetzung von N-(p-Chlorbenzoyl)-3-(p-anisidino)-propionsäure und Sarkosinethylester und entsprechender Aufbereitung N-[N-(p-Chlorbenzoyl)-3-(p-anisidino)-propionyl]-sarkosinethylester (Schmp. 104–106°) erhalten, dessen Verseifung und Aufarbeitung des Reaktionsproduktes die Titelverbindung (Schmp. 129-131°) ergibt.

Beispiel 31
N-[N-(p-Chlorbenzoyl)-3-(p-anisidino)-propionyl]-glycin
$R^1$ = p-Chlorbenzoyl, $R^2$ = p-Methoxyphenyl,
$R^3$ = -H, A = $-CH_2-CH_2-$, B = $-CH_2-$

Analog Beispiel 1 wird bei Einsatz äquivalenter Mengen durch Umsetzung von N-(p-Chlorbenzoyl)-3-(p-anisidino)-propionsäure und Glycinethylester und entsprechender Aufarbeitung N-[N-(p-Chlorbenzoyl)-3-(p-anisidino)-propionyl]-glycinethylester (Schmp. 87–89°) erhalten, dessen Verseifung und Aufarbeitung des Reaktionsproduktes die Titelverbindung (Schmp. 185–187°) ergibt.

Beispiel 32
N-[N-(p-Chlorbenzoyl)-2-(p-anisidino)-acetyl]-sarkosin
$R^1$ = p-Chlorbenzoyl, $R^2$ = p-Methoxyphenyl,
$R^3$ = $-CH_3$, A = B = $-CH_2-$

Analog Beispiel 1 wird bei Einsatz äquivalenter Mengen durch Umsetzung von N-(p-Chlorbenzoyl)-2-(p-anisidino)-essigsäure und Sarkosinethylester (Öl) erhalten, dessen Verseifung und Aufarbeitung des Reaktionsproduktes die Titelverbindung (Schmp. 146–148°) ergibt.

Beispiel 33
N-[N-(p-Chlorbenzoyl)-2-(p-anisidino)-acetyl]-glycin
$R^1$ = p-Chlorbenzoyl, $R^2$ = p-Methoxyphenyl,
$R^3$ = -H, A = B = $-CH_2-$

Analog Beispiel 1 wird bei Einsatz äquivalenter Mengen durch Umsetzung von N-(p-Chorbenzoyl)-2-(p-anisidino)-essigsäure und Glycinethylester und entsprechender Aufarbeitung N-[N-(p-Chlorbenzoyl)-2-(p-anisidino)-acetyl]-glycinethylester (Öl) erhalten, dessen Verseifung und Aufarbeitung des Reaktionsproduktes die Titelverbindung (Schmp. 138–140°) ergibt.

Beispiel 34
N-[N-(p-Chlorbenzoyl)-2-(p-anisidino)-acetyl]-3-aminopropionsäure
$R^1$ = p-Chlorbenzoyl, $R^2$ = p-Methoxyphenyl,
$R^3$ = -H, A = $-CH_2$, B = $-CH_2-CH_2-$

Analog Beispiel 1 wird bei Einsatz äquivalenter Mengen durch Umsetzung von N-(p-Chlorbenzoyl)-2-(p-anisidino)-essigsäure und 3-Aminopropionsäureethylester und entsprechender Aufarbeitung n-[N-(p-Chlorbenzoyl)-2-(p-anisidino)-acetyl]-3-aminopropionsäure-ethylester (Öl) erhalten, dessen Verseifung und Aufarbeitung des Reaktionsproduktes die Titelverbindung (Schmp. 129–131°) ergibt.

Beispiel 35
N-[N-(p-Chlorbenzoyl)-2-(2,6-dimethylanilino)-acetyl]-glycin
$R^1$ = p-Chlorbenzoyl, $R^2$ = 2,6-Dimethylphenyl,
$R^3$ = -H, A = B = $-CH_2-$

Analog Beispiel 1 wird bei Einsatz äquivalenter Mengen durch Umsetzung von N-(p-Chlorbenzoyl)-2-(2,6-dimethylanilino)-essigsäure und Glycinethylester und entsprechender Aufarbeitung N-[N-(p-Chlorbenzoyl)-2-(2,6-dimethylanilino)-acetyl]-glycinethylester (Schmp. 127–129°) erhalten, dessen Verseifung und Aufarbeitung des Reaktionsproduktes die Titelverbindung (Schmp. 200–201°) ergibt.

Beispiel 36
N-[N-(p-Chlorbenzoyl)-2-(2,6-dimethylanilino)-acetyl]-3-aminopropionsäure
$R^1$ = p-Chlorbenzoyl, $R^2$ = 2,6-Dimethylphenyl,
$R^3$ = -H, A = $-CH_2$, B = $-CH_2-CH_2-$

Analog Beispiel 1 wird bei Einsatz äquivalenter Mengen durch Umsetzung von N-(p-Chlorbenzoyl)-2-(2,6-dimethylanilino)-essigsäure und 3-Aminopropionsäureethylester und entsprechender Aufarbeitung N-[N-(p-Chlorbenzoyl)-2-(2,6-dimethylanilino)-acetyl]-3-aminopropionsäure-ethylester (Schmp. 91–92°) erhalten, dessen Verseifung und Aufarbeitung des Reaktionsproduktes die Titelverbindung (Schmp. 154–156°) ergibt.

Beispiel 37
N-[N-(p-Chlorbenzoyl)-4-(2,6-dimethylanilino)-butyryl]-L-prolin
$R^1$ = p-Chlorbenzoyl, $R^2$ = 2,6-Dimethylphenyl,
A = $-CH_2-CH_2-CH_2-$, B = $-CH(R^5)-$,
$R^3 + R^5$ = $-CH_2-CH_2-CH_2-$

Analog Beispiel 1 wird bei Einsatz äquivalenter Mengen durch Umsetzung von N-(p-Chlorbenzoyl)-4-(2,6-dimethylanilino)-buttersäure und L-Prolinethylester und entsprechender Aufarbeitung N-[N-(p-Chlorbenzoyl)-4-(2,6-dimethylanilino)-butyryl]-L-prolinethylester (Öl) erhalten, dessen Verseifung und Aufarbeitung des Reaktionsproduktes die Titelverbindung (Schmp. 129–131°) ergibt.

Beispiel 38
N-[N-(p-Chlorbenzoyl)-2-(2,6-dimethylanilino)-acetyl]-L-prolin
$R^1$ = p-Chlorbenzoyl, $R^2$ = 2,6-Dimethylphenyl,
A = $-CH_2-$, B = $-CH(R^5)-$, $R^3 + R^5$ = $-CH_2-CH_2-CH_2-$

Analog Beispiel 1 wird bei Einsatz äquivalenter Mengen durch Umsetzung von N-(p-Chlorbenzoyl)-2-(2,6-dimethylanilino)-essigsäure und L-Prolinethylester und entsprechender Aufarbeitung N-[N-(p-Chlorbenzoyl)-2-(2,6-dimethylanilino)-acetyl]-L-prolinethylester (Öl) erhalten, dessen Verseifung und Aufarbeitung des Reaktionsproduktes die Titelverbindung (Schmp. 199°) ergibt.

Beispiel 39
N-[N-(p-Chlorbenzoyl)-4-(p-anisidino)-butyryl]-6-(benzhydrylamino)-hexansäure
$R^1$ = p-Chlorbenzoyl, $R^2$ = p-Methoxyphenyl,
$R^3$ = Benzhydryl, A = $-CH_2-CH_2-CH_2-$,
B = $-CH_2-CH_2-CH_2-CH_2-CH_2-$

Analog Beispiel 1 wird bei Einsatz äquivalenter Mengen durch Umsetzung von N-(p-Chlorbenzoyl)-4-(p-anisidino)-buttersäure und 6-(Benzhydrylamino)-hexansäureethylester und entsprechender Aufarbeitung N-[N-(p-Chlorbenzoyl)-4-(p-anisidino)-butyryl]-6-(benzhydrylamino)-hexansäureethylester (Öl) erhalten, dessen Verseifung und Aufarbeitung des Reaktionsproduktes die Titelverbindung als Öl ergibt.

Beispiel 40
N-[N-(Propionyl)-4-(2,6-dimethylanilino)-butyryl]-4-(benzylamino)-buttersäure
$R^1$ = $CH_3-CH_2-CO$, $R^2$ = 2,6-Dimethylphenyl,
$R^3$ = Benzyl, A = B = $-CH_2-CH_2-CH_2-$

Analog Beispiel 1 wird bei Einsatz äquivalenter Mengen durch Umsetzung von N-Propionyl-4-(2,6-dimethylanilino)-buttersäure und 4-(Benzylamino)-buttersäure-ethylester und entsprechender Aufarbeitung N-[N-(Propionyl)-4-(2,6-dimethylanilino)-butyryl]-4-(benzylamino)-buttersäure-ethylester (Öl) erhalten, dessen Verseifung und Aufarbeitung des Reaktionsproduktes die Titelverbindung als Öl ergibt.

Beispiel 41
N-[N-p-Chlorbenzoyl-4-(p-anisidino)-butyryl]-4-(p-anisidino)-buttersäure
$R^1$ = p-Chlorbenzoyl, $R^2$ = $R^3$ = p-Methoxyphenyl,
A = B = $-CH_2-CH_2-CH_2-$

Eine Lösung von 34,8g N-p-Chlorbenzoyl-4-(p-anisidino)-buttersäure in 100ml Benzol wird mit 35,7g Thionylchlorid und einem Tropfen Pyridin versetzt und dann 1 Stunde unter Rückfluss zum Sieden erhitzt. Das Lösungsmittel wird im Vakuum abdestilliert und der Rückstand (35,9g) in 60ml Benzol gelöst. Diese Lösung wird mit einer Lösung von 22,5g 4-(p-Anisidino)-buttersäuremethylester und 13,5g Ethyldiisopropylamin in 150ml Benzol vermischt und 30 Minuten bei Raumtemperatur gerührt. Der ausgefallene Niederschlag wird abfiltriert und das Filtrat eingedampft. Der Eindampfrückstand (56,0g) wird in 100ml Ethanol gelöst und nach Zugabe einer Lösung von 8,5g Kaliumhydroxid in 100ml Ethanol 2 Stunden bei Raumtemperatur gerührt. Das Lösungsmittel wird im Vakuum abdestilliert, der Rückstand in Wasser gelöst und in wässrige Lösung mit verdünnter Salzsäure schwach angesäuert. Das abgeschiedene Produkt wird mit Diethylether extrahiert und der eingedampfte Etherextrakt durch Säulenchromatographie (Adsorbens: Kieselgel, Laufmittel: Chloroform) gereinigt. Aus der Hauptfraktion werden durch Abdampfen des Lösungsmittels 25,9g (48% d.Th.) der Titelverbindung als zähes Öl erhalten. Dieses kristallisiert nach längerem Stehen (10 Wochen) in Essigsäureethylester-Petrolether. Die Kristalle schmelzen bei 62–64°.

Beispiel 42
N-[N-(p-Chlorbenzoyl-4-(1.1.3.3-tetramethylbutylamino)-butyryl]-4-(p-anisidino)-buttersäure
$R^1$ = p-Chlorbenzoyl, $R^2$ = $-C(CH_3)_2-CH_2-C(CH_3)_3$,
$R^3$ = p-Methoxyphenyl, A = B = $-CH_2-CH_2-CH_2-$

Analog Beispiel 1 wird bei Einsatz äquivalenter Mengen durch Umsetzung von N-p-Chlorbenzoyl-4-[(1.1.3.3-tetramethylbutyl)-amino]-buttersäure und 4-(p-Anisidino)-buttersäure-methylester und entsprechende Aufarbeitung, Lösen des Eindampfrückstandes in Ethanol, Versetzen mit ethanolischer Kaliumhydroxidlösung, 12 Stunden

Rühren bei Raumtemperatur und Aufarbeitung die Titelverbindung erhalten.

Beispiel 43
N-[N-p-Fluorbenzoyl-4-(1.1.3.3-tetramethylbutylamino)-butyryl]-L-prolin
$R^1$ = p-Fluorbenzoyl, $R^2$ = $-C(CH_3)_2-CH_2-C(CH_3)_3$,
A = $-CH_2-CH_2-CH_2-$, B = $-CH(R^5)$,
$R^3 + R^5 = -CH_2-CH_2-CH_2-$

Analog Beispiel 1 wird bei Einsatz äquivalenter Mengen durch Umsetzung von N-p-Fluorbenzoyl-4-[(1.1.3.3-tetramethylbutyryl)-amino]-buttersäure und L-Prolinethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 44
N-[N-p-Chlorbenzoyl-4-(tert.-butylamino)-butyryl]-L-serin
$R^1$ = p-Chlorbenzoyl, $R^2$ = $-C(CH_3)_3$, $R^3$ = -H, A = $-CH_2-CH_2-CH_2-$, B = $-CH(R^5)$, $R^5$ = $-CH_2-OH$

Analog Beispiel 1 wird bei Einsatz äquivalenter Mengen durch Umsetzung von N-p-Chlorbenzoyl-4-(tert.-butylamino)-buttersäure und L-Serinethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 45
N-[N-3,4,5-Trimethoxybenzoyl-6-(tert.-butylamino)-hexanoyl]-4-(p-anisidino)-buttersäure
$R^1$ = 3,4,5-Trimethoxybenzoyl, $R^2$ = $-C(CH_3)_3$,
$R^3$ = p-Methoxyphenyl, A = $-CH_2-CH_2-CH_2-CH_2-CH_2-$,
B = $-CH_2-CH_2-CH_2-$

Analog Beispiel 1 wird bei Einsatz äquivalenter Mengen durch Umsetzung von N-3,4,5-Trimethoxybenzoyl-6-(tert.-butylamino)-capronsäure und γ-(p-Anisidino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 46
N-[N-p-Chlorbenzoyl-4-(1,1-dimethylpropylamino)-butyryl]-4-(2,6-diethylanilino)-buttersäure
$R^1$ = p-Chlorbenzoyl, $R^2$ = $-C(CH_3)_2-CH_2-CH_3$,
$R^3$ = 2,6-Diethylphenyl, A = B = $-CH_2-CH_2-CH_2-$

Analog Beispiel 1 wird bei Einsatz äquivalenter Mengen durch Umsetzung von N-p-Chlorbenzoyl-4-[(1,1-dimethylpropyl)-amino]-buttersäure und γ-(2,6-Diethylanilino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 47
N-[N-2,4-Dichlorbenzoyl-4-(1.1-dimethylpropylamino)-butyryl]-4-(p-anisidino)-buttersäure
$R^1$ = 2,4-Dichlorbenzoyl, $R^2$ = $-C(CH_3)_2-CH_2-CH_3$,
$R^3$ = p-Methoxyphenyl, A = B = $-CH_2-CH_2-CH_2-$

Analog Beispiel 1 wird bei Einsatz äquivalenter Mengen durch Umsetzung von N-2,4-Dichlorbenzoyl-4-[(1.1-dimethylpropyl)-amino]-buttersäure und γ-(p-Anisidino)-buttersäuremethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 48
N-[N-n-Butyryl-4-(1.1-dimethylpropylamino)-butyryl]-4-(2-methyl-3-butin-2-yl-amino)-buttersäure
$R^1$ = $CH_3-CH_2-CH_2-CO$, $R^2$ = $-C(CH_3)_2-CH_2-CH_3$,
$R^3$ = $-C(CH_3)_2-C{\equiv}CH$, A = B = $-CH_2-CH_2-CH_2-$

Analog Beispiel 1 wird bei Einsatz äquivalenter Mengen durch Umsetzung von N-n-Butyryl-4-[(1.1-dimethylpropyl)-amino]-buttersäure und 4-[(2-Methyl-3-butin-2-yl)-amino]-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 49
N-[N-p-Chlorbenzoyl-4-(1-ethinyl-cyclohexyl-1-amino)-butyryl]-4-(3-ethyl-1-pentin-3-yl-amino)-buttersäure
$R^1$ = p-Chlorbenzoyl, $R^2$ = 1-Ethinylcyclohexyl-1,
$R^3$, $-(C_2H_5)_2-C{\equiv}CH$, A = B = $-CH_2-CH_2-CH_2-$

Analog Beispiel 1 wird bei Einsatz äquivalenter Mengen durch Umsetzung von N-p-Chlorbenzoyl-4-[(ethinyl-cyclohexyl-1)-amino]-buttersäure und 4-[(3-Ethyl-1-pentin-3-yl)-amino]-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 50
N-[N-p-Chlorbenzoyl-4-(1-n-propylcyclohexyl-1-amino)-butyryl]-4-(1-ethinyl-cyclohexyl-1-amino)-buttersäure
$R^1$ = p-Chlorbenzoyl, $R^2$ = 1-n-Propylcyclohexyl-1,
$R^3$ = 1-Ethinylcyclohexyl-1, A = B = $-CH_2-CH_2-CH_2-$

Analog Beispiel 1 wird bei Einsatz äquivalenter Mengen durch Umsetzung von N-p-Chlorbenzoyl-4-[(1-propyl-cyclohexyl-1)-amino]-buttersäure und 4-[(1-Ethinyl-cyclohexyl-1)-amino]-buttersäure-ethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 51
N-[N-p-Chlorbenzoyl-4-(1-n-butylcyclopentyl-1-amino)-butyryl]-4-aminobuttersäure

$R^1$ = p-Chlorbenzoyl, $R^2$ = 1-n-butylcyclopentyl-1, $R^3$ = -H, A = B = $-CH_2-CH_2-CH_2-$

Analog Beispiel 1 wird bei Einsatz äquivalenter Mengen durch Umsetzung von N-p-Chlorbenzoyl-4-[(1-n-butyl-cyclopentyl-1)-amino]-buttersäure und 4-Aminobuttersäuremethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 52

N-[N-p-Chlorbenzoyl-4-(adamantylamino)-butyryl]-3-aminopropionsäure

$R^1$ = p-Chlorbenzoyl, $R^2$ = 1-Adamantyl, $R^3$ = -H, A = $-CH_2-CH_2-CH_2-$, B = $-CH_2-CH_2-$

Analog Beispiel 1 wird bei Einsatz äquivalenter Mengen durch Umsetzung von N-p-Chlorbenzoyl)-4-(1-adamantyl)-aminobuttersäure und 3-Aminopropionsäure-ethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 53

N-[N-p-Chlorbenzoyl-4-(cyclooctylamino)-butyryl]-L-alanin

$R^1$ = p-Chlorbenzoyl, $R^2$ = Cyclooctyl, $R^3$ -H, A = $-CH_2-CH_2-CH_2-$, B = $-CH(R^5)-$, $R^5$ = $CH_3$

Analog Beispiel 1 wird bei Einsatz äquivalenter Mengen durch Umsetzung von N-p-Chlorbenzoyl-4-cyclooctylamino-buttersäure und L-Alaninmethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 54

N-[Acetyl-4-(benzhydrylamino)-butyryl]-6-(benzhydrylamino)-capronsäure

$R^1$ = $-CH_3-CO$, $R_2$ = $R^3$ = Benzhydryl, A = $-CH_2-CH_2-CH_2-$, B = $-CH_2-CH_2-CH_2-CH_2-CH_2-$

Analog Beispiel 1 wird bei Einsatz äquivalenter Mengen durch Umsetzung von N-Acetyl-4-benzhydrylamino-buttersäure und 6-Benzhydrylaminocapronsäure-ethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 55

N-[N-p-Chlorbenzoyl-4-(benzhydrylamino)-butyryl]-5-(benzhydrylamino)-valeriansäure

$R^1$ = p-Chlorbenzoyl, $R^2$ = $R^3$ = Benzhylryl, A = $-CH_2-CH_2-CH_2-$, B = $-CH_2-CH_2-CH_2-CH_2-$

Analog Beispiel 1 wird bei Einsatz äquivalenter Mengen durch Umsetzung von N-p-Chlorbenzoyl-4-benzhydrylamino-buttersäure und 5-(Benzhydrylamino)-valeriansäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 56

N-[N-p-Chlorbenzoyl-4-(1-phenylethylamino)-butyryl]-4-(benzylamino)-buttersäure

$R^1$ = p-Chlorbenzoyl, $R^2$ = 1-Phenylethyl, $R^3$ = Benzyl, A = B = $-CH_2-CH_2-CH_2-$

Analog Beispiel 1 wird bei Einsatz äquivalenter Mengen durch Umsetzung von N-p-Chlorbenzoyl-4-(1-phenylethyl-amino)-buttersäure und 4-Benzylaminobuttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 57

N-[N-p-Chlorbenzoyl-6-(1-phenylethylamino)-hexanoyl]-4-(benzyl-amino)-buttersäure

$R^1$ = p-Chlorbenzoyl, $R^2$ = 1-Phenylethyl, $R^3$ = Benzyl, A = $-CH_2-CH_2-CH_2-CH_2-CH_2-$, B = $-CH_2-CH_2-CH_2-$

Analog Beispiel 1 wird bei Einsatz äquivalenter Mengen durch Umsetzung von N-p-Chlorbenzoyl-6-(1-phenylethylamino)-capronsäure und 4-Benzylaminobuttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 58

N-[N-p-Chlorbenzoyl-4-(homoveratrylamino)-butyryl]-4-aminobuttersäure

$R^1$ = p-Chlorbenzoyl, $R^2$ = Homoveratryl, $R^3$ = -H, A = B = $-CH_2-CH_2-CH_2-$

Analog Beispiel 1 wird bei Einsatz äquivalenter Mengen durch Umsetzung von N-p-Chlorbenzoyl-4-homoveratrylamino-buttersäure und 4-Aminobuttersäure-methylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 59

N-[N-p-Chlorbenzoyl-4-(1,2-diphenylethylamino)-butyryl]-3-aminopropionsäure

$R^1$ = p-Chlorbenzoyl, $R^2$ = 1,2-Diphenylethyl, $R^3$ = -H, A = $-CH_2-CH_2-CH_2-$, B = $-CH_2-CH_2-$

Analog Beispiel 1 wird bei Einsatz äquivalenter Mengen durch Umsetzung von N-p-Chlorbenzoyl-4-[(1,2-diphenylethyl)-amino]-buttersäure und 3-Aminopropionsäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 60
N-[N-m-Trifluormethylbenzoyl-4-(1.1.3.3-tetramethylbutylamino)-butyryl]-3-aminopropionsäure
$R^1$ = m-Trifluormethylbenzoyl,
$R^2$ = $-C(CH_3)_2-CH_2-C(CH_3)_3$, $R^3$ = -H,
A = $-CH_2-CH_2-CH_2-$, B = $-CH_2-CH_2-$

Analog Beispiel 1 wird bei Einsatz äquivalenter Mengen durch Umsetzung von N-m-Trifluormethyl-benzoyl-4-[(1.1.3.3-tetramethylbutyl)-amino]-buttersäure und 3-Aminopropionsäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 61
N-[N-Crotonoyl-4-(1.1.3.3-tetramethylbutyl-amino)-butyryl]-6-(benzhydrylamino)-capronsäure
$R^1$ = $CH_3-CH=CH-CO$, $R^2$ = $-C(CH_3)_2-CH_2-C(CH_3)_3$,
$R^3$ = Benzhydryl, A = $-CH_2-CH_2-CH_2-$,
B = $-CH_2-CH_2-CH_2-CH_2-CH_2-$,

Analog Beispiel 1 wird bei Einsatz äquivalenter Mengen durch Umsetzung von N-Crotonoyl-4-[(1.1.3.3-tetramethylbutyl)-amino]-buttersäure und 6-(Benzhydrylamino)-hexansäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 62
N-[N-Propionyl-4-(benzhydrylamino)-butyryl]-5-(benzhydrylamino)-valeriansäure
$R^1$ = $-CH_3-CH_2-CO-$, $R^2$ = $R^3$ = benzhydryl,
A = $-CH_2-CH_2-CH_2-$, B = $-CH_2-CH_2-CH_2-CH_2-$

Analog Beispiel 1 wird bei Einsatz äquivalenter Mengen durch Umsetzung von N-Propionyl-4-benzhydrylamino-buttersäure und 5-Benzhydrylamino-valeriansäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 63
N-[N-5-Chlor-2-methoxybenzoyl-4-(benzhydrylamino)-butyryl]-6-(benzhydrylamino)-capronsäure
$R^1$ = 5-Chlor-2-methoxybenzoyl, $R^2$ = $R^3$ = Benzhydryl, A = $-CH_2-CH_2-CH_2-$,
B = $-CH_2-CH_2-CH_2-CH_2-CH_2-$

Analog Beispiel 1 wird bei Einsatz äquivalenter Mengen durch Umsetzung von N-(5-Chlor-2-methoxy-benzoyl)-4-benzhydrylamino-buttersäure und 6-(Benzhydrylamino)-hexansäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 64
N-[N-Acetyl-6-(benzhydrylamino)-hexanoyl]-L-serin
$R^1$ = $CH_3-CO-$, $R^2$ = Benzhydryl, $R^3$ = -H, A = $-CH_2-CH_2-CH_2-CH_2-CH_2-$, B = $-CH(R^5)-$, $R^5$ = $-CH_2-OH$

Analog Beispiel 1 wird bei Einsatz äquivalenter Mengen durch Umsetzung von N-Acetyl-6-benzhydrylamino-capronsäure und L-Serinethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 65
N-[N-Isobutyryl-6-(benzhydrylamino)-hexanoyl]-6-(benzhydrylamino)-capronsäure
$R^1$ = $CH(CH_3)_2-CO$, $R^2$ = $R^3$ = Benzhydryl,
A = B = $-CH_2-CH_2-CH_2-CH_2-CH_2-$,

Analog Beispiel 1 wird bei Einsatz äquivalenter Mengen durch Umsetzung von N-Isobutyryl-6-benzhydrylamino-capronsäure und 6-Benzhydrylamino-capronsäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 66
N-[N-Acetyl-5-(benzhydrylamino)-pentanoyl]-5-(benzhydrylamino)-valeriansäure
$R^1$ = $CH_3-CO$, $R^2$ = $R^3$ = Benzhydryl,
A = B = $-CH_2-CH_2-CH_2-CH_2-$

Analog Beispiel 1 wird bei Einsatz äquivalenter Mengen durch Umsetzung von N-Acetyl-5-benzhydrylamino-valeriansäure und 5-Benzhydrylamino-valeriansäure-ethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 67
N-[N-Crotonoyl-5-(benzhydrylamino)-pentanoyl]-3-aminopropionsäure
$R^1$ = $CH_3-CH=CH-CO$, $R^2$ = Benzhydryl, $R^3$ = -H,
A = $-CH_2-CH_2-CH_2-CH_2-$, B = $-CH_2-CH_2-$

Analog Beispiel 1 wird bei Einsatz äquivalenter Mengen durch Umsetzung von N-Crotonoyl-5-benzhydrylamino-valeriansäure und 3-Aminopropionsäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 68
N-[N-Benzoyl-4-(n-butylamino)-butyryl]-6-(benzhydrylamino)-capronsäure

$R^1$ = Benzoyl, $R^2$ = $-CH_2-CH_2-CH_2-CH_3$, $R^3$ = Benzhydryl, A = $-CH_2-CH_2-CH_2-$, B = $-CH_2-CH_2-CH_2-CH_2-CH_2-$

Analog Beispiel 1 wird bei Einsatz äquivalenter Mengen durch Umsetzung von N-Benzoyl-4-n-butylamino-buttersäure und 6-(Benzhydrylamino)-capronsäure-ethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 69

N-[N-p-Chlorbenzoyl-5-(n-butylamino)-pentanoyl]-5-(benzhydrylamino)-valeriansäure

$R^1$ = p-Chlorbenzoyl, $R^2$ = $-CH_2-CH_2-CH_2-CH_3$, $R^3$ = Benzhydryl, A = B = $-CH_2-CH_2-CH_2-CH_2-$

Analog Beispiel 1 wird bei Einsatz äquivalenter Mengen durch Umsetzung von N-p-Chlorbenzoyl-5-(n-butylamino)-valeriansäure und 5-(Benzhydrylamino)-valeriansäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 70

N-[N-p-Chlorbenzoyl-4-(benzylamino)-butyryl]-4-(benzhydrylamino)-buttersäure

$R^1$ = p-Chlorbenzoyl, $R^2$ = Benzyl, $R^3$ = Benzhydryl, A = B = $-CH_2-CH_2-CH_2-$

Analog Beispiel 1 wird bei Einsatz äquivalenter Mengen durch Umsetzung von N-p-Chlorbenzoyl-4-benzylamino-buttersäure und 4-(Benzhydrylamino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 71

N-[N-p-Chlorbenzoyl-5-(benzylamino)-pentanoyl]-6-(benzhydrylamino)-capronsäure

$R^1$ = p-Chlorbenzoyl, $R^2$ = Benzyl, $R^3$ = Benzhydryl, A = $-CH_2-CH_2-CH_2-CH_2-$, B = $-CH_2-CH_2-CH_2-CH_2-CH_2-$

Analog Beispiel 1 wird bei Einsatz äquivalenter Mengen durch Umsetzung von N-p-Chlorbenzoyl-5-benzylamino-valeriansäure und 6-(Benzhydrylamino)-capronsäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 72

N-[N-p-Methoxybenzoyl-L-phenylalanyl]-4-(benzhydrylamino)-buttersäure

$R^1$ = p-Methoxybenzoyl, $R^2$ = -H, $R^3$ = Benzhydryl, A = $-CH(R^4)$, $R^4$ = Benzyl, B = $-CH_2-CH_2-CH_2-$

Analog Beispiel 1 wird bei Einsatz äquivalenter Mengen durch Umsetzung von N-(p-Methoxybenzoyl)-L-phenylalanin und 4-(Benzhydrylamino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 73

N-[N-p-Chlorbenzoyl-L-methionyl]-4-(benzylamino)-buttersäure

$R^1$ = p-Chlorbenzoyl, $R^2$ = -H, $R^3$ = Benzyl, A = $-CH(R^4)$, $R^4$ = $CH_3-S-CH_2-CH_2-$, B = $-CH_2-CH_2-CH_2-$

Analog Beispiel 1 wird bei Einsatz äquivalenter Mengen durch Umsetzung von N-p-Chlorbenzoyl-L-methionin und 4-(Benzylamino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 74

N-[N-2-Furoyl-2-(p-anisidino)-propionyl]-4-(1-phenylethylamino)-buttersäure

$R^1$ = 2-Furoyl, $R^2$ = p-Methoxyphenyl, $R^3$ = 1-Phenylethyl, A = $-CH(R^4)$, $R^4$ = $-CH_3$, B = $-CH_2-CH_2-CH_2-$

Analog Beispiel 1 wird bei Einsatz äquivalenter Mengen durch Umsetzung von N-(2-Furoyl)-2-(p-anisidino)-propionsäure und 4-(1-Phenylethylamino)-buttersäureethylester und entsprechende Aufarbeitung, Lösen des Eindampfrückstandes in Ethanol, Versetzen mit ethanolischer Kaliumhydroxidlösung, 12 Stunden Rühren bei Raumtemperatur und Aufarbeitung die Titelverbindung erhalten.

Beispiel 75

Ampullen mit 600 mg N-[N-Acetyl-2-(p-anisidino)-acetyl]-4-(p-anisidino)-buttersäure, Chargengrösse 250 kg

25 kg 1,2-Propylenglykol und 150 kg Aqua bidestillata werden vorgelegt, 15 kg N-[N-Acetyl-2-(p-anisidino)-acetyl]-4-(p-anisidino)-buttersäure werden zugegeben und anschliessend wird langsam, unter Rühren, Natronlauge (10 Gew.-% NaOH, ca. 15 kg) zugegeben. Wenn sich alles gelöst hat, wird mit Natronlauge der pH auf 7,5–8,0 eingestellt. 0,0625 kg Natriumpyrosulfit werden zugegeben und die Mischung wird gerührt, bis sich alles gelöst hat. Mit Aqua bidestillata wird auf 250 kg aufgefüllt. Die Lösung wird in 10 ml Ampullen abgefüllt und bei 120° 30 Minuten im Autoklaven sterilisiert.

Beispiel 76

Ampullen mit 600 mg N-[N-p-Chlorbenzoyl-4-(2,6-dimethylanilino)-butyryl]-4-(p-anisidino)-buttersäure, Chargengrösse 250 kg

50 kg 1,2-Propylenglykol und 150 kg Aqua bidestillata werden vorgelegt. Dann werden unter

Rühren 15kg N-[N-p-Chlorbenzoyl-4-(2,6-dimethylanilino)-butyryl]-4-(p-anisidino)-buttersäure zugegeben. Anschliessend werden zunächst 15kg Natronlauge (10 Gew.-% NaOH) hinzugefügt und die Mischung dann auf einen pH von 7,5 bis 8,0 eingestellt. Mit Aqua bidestillata wird auf 250kg ergänzt. Die Lösung wird in 10ml-Ampullen abgefüllt und 30 Minuten bei 120° im Autoklaven sterilisiert.

Beispiel 77

Tabletten mit 50mg N-[N-p-Chlorbenzoyl-4-(2,6-dimethylphenyl)-butyryl]-4-(p-anisidino)-buttersäure

25kg N-[N-p-Chlorbenzoyl-4-(2,6-dimethylphenyl)-butyryl]-4-(p-anisidino)-buttersäure, 35kg Milchzucker und 26kg Maisstärke werden mit 2,5kg Polyvinylpyrrolidon (Molekulargewicht ca. 25.000) in ungefähr 6l Wasser granuliert. Das Granulat wird durch ein Sieb von 1,25mm Maschenweite gesiebt und nach dem Trocknen werden 8kg Carboxymethylcellulose, 2kg Talkum und 1kg Magnesiumstearat zugegeben. Man verpresst das trockene Granulat zu Tabletten von 8mm Durchmesser, 250mg Gewicht und einer Härte von 5–6kg.

In ähnlicher Weise werden Tabletten mit N-[N-Acetyl-2-(p-anisidino)-acetyl]-4-(p-anisidino)-buttersäure bzw. N-[N-p-Chlorbenzoyl-4-(2,6-dimethylanilino)-butyryl]-4-aminobuttersäure hergestellt.

Beispiel 78

Tabletten mit 100mg N-[N-p-Chlorbenzoyl-methionyl]-4-(p-anisidino)-buttersäure

40kg N-[N-p-Chlorbenzoyl-methionyl]-4-(p-anisidino)-buttersäure, 24kg Milchzucker und 16kg Maisstärke werden mit 4kg Polyvinylpyrrolidon (Molekulargewicht 25.000) in ungefähr 5,5l Wasser granuliert und durch ein Sieb von 1,25mm Maschenweite gepresst. Nach dem Trocknen werden 10kg Carboxymethylcellulose, 4kg Talkum und 2kg Magnesiumstearat zugegeben. Auf einer Exzentermaschine wird das Granulat zu Tabletten von 9mm Durchmesser 250mg Gewicht und einer Härte von 4–5kg verpresst.

Beispiel 79

Tabletten mit 300mg N-[N-p-Chlorbenzoyl-4-(p-anisidino)-butyryl]-4-(p-anisidino)-buttersäure

60kg N-[N-p-Chlorbenzoyl-4-(p-anisidino)-butyryl]-4-(p-anisidino)-buttersäure, 12kg Milchzucker und 8kg Maisstärke werden mit 4kg Polyvinylpyrrolidon (Molekulargewicht ca. 25.000) in ungefähr 6l Wasser granuliert und durch ein Sieb von 1,25mm Maschenweite gepresst. Nach dem Trocknen werden 10kg Carboxymethylcellulose, 4kg Talkum und 2kg Magnesiumstearat zugegeben. Auf einem Rundläufer wird das Granulat zu Tabletten von 11mm Durchmesser, 500mg Gewicht und einer Härte von 6–7kg verpresst.

Beispiel 80

10.000 Kapseln mit einem Wirkstoffgehalt von 50mg werden aus folgenden Bestandteilen hergestellt: 500g N-[N-Acetyl-3-(2,6-dimethylanilino)-propionyl]-4-(2-ethyl-6-methylanilino)-buttersäure, 495g mikrokristalline Cellulose und 5g amorphe Kieselsäure werden gut gemischt und in Hartgelatinekapseln Grösse 4 abgefüllt.

Pharmakologie

Die N-substituierten ω-Aminoalkanoyl-ω-aminoalkansäuren üben einen starken Einfluss auf die Pankreassekretion narkotisierter Ratten aus und beeinflussen die Gallesekretion narkotisierter Ratten, wobei sie sich bekannten Handelspräparaten, z.B. Piprozolin, überlegen erweisen.

In den anschliessenden Tabellen werden die untersuchten Verbindungen durch eine laufende Nummer gekennzeichnet, die wie folgt zugeordnet ist:

| Lfd. Nr. | Name der Verbindung |
|---|---|
| 1 | Piprozolin |
| 2 | N-[N-(p-Chlorbenzoyl)-4-(2,6-dimethylanilino)-butyryl]-sarkosin |
| 3 | N-[N-(p-Chlorbenzoyl)-2-(p-anisidino)-acetyl]-sarkosin |
| 4 | N-[N-(p-Chlorbenzoyl)-2-(p-anisidino)-acetyl]-glycin |
| 5 | N-[N-(p-Chlorbenzoyl)-2-(p-anisidino)-acetyl]-3-aminopropionsäure |
| 6 | N-[N-(p-Chlorbenzoyl)-2-(2,6-dimethylanilino)-acetyl]-glycin |
| 7 | N-[N-(p-Chlorbenzoyl)-2-(2,6-dimethylanilino)-acetyl]-3-aminopropionsäure |
| 8 | N-[N-Acetyl-2-(p-anisidino)-acetyl]-4-(p-anisidino)-buttersäure |
| 9 | N-[N-(p-Chlorbenzoyl)-2-(p-anisidino)-acetyl]-4-(p-anisidino)-buttersäure |
| 10 | N-[N-(p-Chlorbenzoyl)-3-(p-anisidino)-propionyl]-4-(p-anisidino)-buttersäure |
| 11 | N-[N-(p-Chlorbenzoyl)-4-(2,6-dimethylanilino)-butyryl]-4-(2,6-dimethylanilino)-buttersäure |
| 12 | N-[N-(p-Chlorbenzoyl)-4-(p-anisidino)-butyryl]-4-(2,6-dimethylanilino)-buttersäure |
| 13 | N-[N-p-Chlorbenzoyl-4-(2,6-dimethylanilino)-butyryl-4-(p-anisidino)-buttersäure |
| 14 | N-[N-p-Chlorbenzoyl-4-(2,6-dimethylanilino)-butyryl]-4-aminobuttersäure |
| 15 | N-[N-(3,4,5-Trimethoxybenzoyl)-4-(2,6-dimethylanilino)-butyryl]-4-aminobuttersäure |
| 16 | N-[N-Acetyl-2-(p-anisidino)-propionyl]-4-(p-anisidino)-buttersäure |
| 17 | N-[N-(2-Furoyl)-2-(p-anisidino)-propionyl]-4-(p-anisidino)-buttersäure |
| 18 | N-[N-(p-Chlorbenzoyl)-2-(p-anisidino)-propionyl]-4-(p-anisidino)-buttersäure |
| 19 | N-[N-(m-Trifluormethylbenzoyl)-2-(p-anisidino)-propionyl]-4-(p-anisidino)-buttersäure |
| 20 | N-[N-Acetyl-3-(2,6-dimethylanilino)-propionyl]-4-(p-anisidino)-buttersäure |
| 21 | N-[N-Acetyl-3-(2,6-dimethylanilino)-propionyl]-4-(2,6-dimethylanilino)-buttersäure |
| 22 | N-[N-Acetyl-3-(2,6-dimethylanilino)-propionyl]-4-(2-ethyl-6-methylanilino)-buttersäure |

23 N-[N-(p-Chlorbenzoyl)-methionyl]-4-(p-anisidino)-buttersäure
24 N-[N-(p-Methoxybenzoyl)-phenylalanyl]-4-(p-anisidino)-buttersäure
25 N-[N-(p-Chlorbenzoyl)-3-(2,6-dimethylanilino)-propionyl]-4-(p-anisidino)-buttersäure

In Tabelle I werden Untersuchungen der Pankreassekretion von narkotisierten Ratten nach intraduodenaler Applikation ($ED_{50}$) und die letale Wirkung an der Maus ($LD_{50}$) nach intraperitonealer Applikation von Vertretern der erfindungsgemässen Verbindungen sowie der therapeutische Quotient (TQ = $LD_{50}/ED_{50}$) wiedergegeben.

Tabelle I
Pankreassekretion, Toxizität und therapeutischer Quotient

| Lfd. Nr. | Toxizität $LD_{50}$ [mg/kg] (Maus, i.p.) | Pankreassekretion $ED_{50}$* [mg/kg] (Ratte, i.d.) | TQ [$LD_{50}/ED_{50}$] |
|---|---|---|---|
| 1 | 1070** | 35 | 31 |
| 2 | 700 | 10 | 70 |
| 3 | ≫1000 | 15 | ≫ 67 |
| 4 | >1000 | 15 | > 67 |
| 5 | 1300 | 25 | 52 |
| 6 | 400 | ~ 5 | ~ 80 |
| 7 | 450 | ≲ 1 | ≳ 450 |
| 8 | 1200 | 1 | 1200 |
| 9 | 420 | 3 | 140 |
| 10 | 220 | ~ 1 | ~ 220 |
| 11 | 130 | 1 | 130 |
| 12 | 800 | 2 | 400 |
| 13 | 150 | 0,8 | 188 |
| 14 | 400 | 0,4 | 1000 |
| 15 | 800 | ~ 1 | ~ 800 |
| 17 | 750 | 5 | 150 |
| 18 | > 250 | 5 | ≲ 50 |
| 20 | 600 | 7 | 86 |
| 22 | 430 | 3 | 143 |
| 24 | 300 | 6 | 50 |

* $ED_{50}$ = Dosis, die eine Steigerung der Pankreassekretion (Flüssigkeitsvolumen; 30-min-Fraktion) um maximal 50% bewirkt.
** $LD_{50}$ [p. o.]. zit. aus Herrmann et al., Arzneimittel-Forschung 27 (1977), 467.

In Tabelle II werden Untersuchungen der Gallesekretion (Cholerese) von narkotisierten Ratten nach intraduodenalen Applikationen ($ED_{50}$) und die letale Wirkung an der Maus ($LD_{50}$) nach intraperitonealer Applikation von Vertretern der erfindungsgemässen Verbindungen sowie der therapeutische Quotient (TQ = $LD_{50}/ED_{50}$) wiedergegeben.

Tabelle II
Gallesekretion, Toxizität und therapeutischer Quotient

| Lfd. Nr. | Toxizität $LD_{50}$ [mg/kg] (Maus, i.p.) | Gallesekretion $ED_{50}$*** [mg/kg] (Ratte, i.d.) | TQ [$LD_{50}/ED_{50}$] |
|---|---|---|---|
| 1 | 1070** | 40 | 27 |
| 7 | 450 | 10 | 45 |
| 11 | 130 | 3 | 43 |
| 12 | 800 | 14 | 57 |
| 16 | 600 | 10 | 60 |
| 17 | 750 | 11 | 68 |
| 18 | ≲ 250 | 7 | ≲ 36 |
| 19 | 130 | ~ 1 | ~130 |
| 20 | 600 | 7 | 86 |
| 21 | 280 | ~ 1 | ~280 |
| 22 | 430 | 1,3 | 331 |
| 23 | 700 | 2,5 | 280 |
| 24 | 300 | 6 | 50 |
| 25 | 60 | ~ 1 | ~ 60 |

*** $ED_{50}$ = Dosis, die eine Steigerung der Gallesekretion (Flüssigkeitsvolumen; 30-min-Fraktion) um maximal 50% bewirkt.
** $LD_{50}$ [p. o.]. zit. aus Herrmann et al., Arzneimittel-Forschung 27 (1977), 467.

Die Ermittlung der pharmakologischen Eigenschaften erfolgte nach folgenden Methoden:

Einfluss auf die Pankreas- und Gallesekretion der narkotisierten Ratte
Versuchsdurchführung:

Männliche Sprague-Dawley-Ratten (250–300 g Körpergewicht) werden mit 1,2 g/kg Urethan i. m. narkotisiert. Dann wird die Bauchhöhle medial geöffnet der Ductus choledochus kurz oberhalb seiner Mündung in das Duodenum sowie nahe der Leberpforte ligiert, beide Gangabschnitte werden leberwärts katheterisiert.

Da bei der Ratte alle Pankreaskanäle in den mittleren Abschnitt des Ductus choledochus münden, lassen sich auf diese Weise aus dem distalen (ligierten) Gangabschnitt das Pankreassekret und aus dem proximalen Teil des Ductus choledochus die Galle getrennt ableiten.

Die sezernierten Mengen an Pankreas- und Gallensaft werden in 30 min. Abständen während 2 Stunden vor bis 3 Stunden nach der intraduodenalen (V. jugularis externa) Verabreichung der zu prüfenden Verbindungen (verabreichtes Flüssigkeitsvolumen 5 ml/kg) gemessen.

Die Körpertemperatur der Tiere wird mittels Heizkissen und Bestrahlung auf 36 bis 38°C gehalten; die Temperaturkontrolle erfolgt rectal.

Auswertung:

Die Flüssigkeitsvolumina der 30-Min.-Fraktionen nach der Substanzgabe werden jeweils auf die vor der Substanzapplikation ausgeschiedenen Galle- bzw. Pankreassaftmenge (= 100%, Mittel-

wert aus den beiden letzten Messungen) bezogen. Die maximale prozentuale Steigerung der Pankreas- bzw. Gallesekretion wird in Abhängigkeit von der Dosis dargestellt und daraus die $ED_{50}$ durch Interpolation ermittelt.

Bestimmung der Toxizität:

Die Toxizitätsuntersuchungen werden an weiblichen NMRI-Mäusen (Körpergewicht 22–26 g) durchgeführt. Die Tiere (5 Tiere pro Dosis) erhalten Futter und Wasser ad libitum. Verschiedene Dosierungen der Substanzen werden intraperitoneal verabreicht. Die Beobachtungsdauer beträgt 14 Tage. Die $DL_{50}$, das heisst die Dosis, bei der 50% der Tiere sterben, wird aus der Dosiswirkungskurve graphisch ermittelt.

## Pantentansprüche

Anspruch 1

N-substituierte ω-Aminoalkanoyl-ω-aminoalkansäuren der allgemeinen Formel I

$$R^1-\underset{R^2}{\underset{|}{N}}-A-CO-\underset{R^3}{\underset{|}{N}}-B-COOH \qquad (I),$$

worin

$R^1$ einen Alkanoylrest oder Alkenoylrest mit 2 bis 5 Kohlenstoffatomen, einen Furoylrest oder einen Benzoylrest

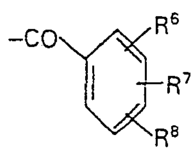

bedeutet,

$R^2$ ein Wasserstoffatom, den Rest $-C(R^9)(R^{10})(R^{11})$ oder einen Phenylrest

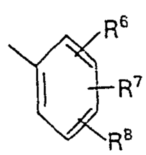

bedeutet,

$R^3$ ein Wasserstoffatom, einen Alkylrest mit 1 bis 5 Kohlenstoffatomen, den Rest $-C(R^9)(R^{10})(R^{11})$ oder einen Phenylrest

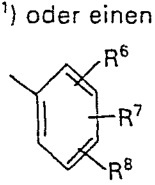

bedeutet,

wobei $R^2$ und $R^3$ nicht gleichzeitig ein Wasserstoffatom und nicht gleichzeitig einen geradkettigen Alkylrest darstellen,

A eine $-(CH_2)_m$-Gruppe oder eine $-CH(R^4)$-Gruppe bedeutet,

B eine $-(CH_2)_n$-Gruppe oder eine $-CH(R^5)$-Gruppe bedeutet,

m und n gleich oder verschieden sind und eine positive ganze Zahl von 1 bis 5 bedeuten,

$R^4$ und $R^5$ gleich oder verschieden sind und eine Methylgruppe, eine Benzylgruppe, eine Hydroxymethylgruppe, eine 2-Hydroxyethylgruppe, eine Methylmercaptomethylgruppe oder eine 2-Methylmercaptoethylgruppe bedeuten oder (unter der Voraussetzung, dass $R^2$ kein Wasserstoffatom darstellt)

$R^3$ und $R^5$ gemeinsam eine Trimethylgruppe bedeuten,

$R^6$, $R^7$ und $R^8$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

$R^9$ ein Wasserstoffatom, eine Alkylgruppe mit 1 bis 5 Kohlenstoffatomen, eine Alkenylgruppe oder eine Alkinylgruppe mit 2 bis 5 Kohlenstoffatomen,

$R^{10}$ ein Wasserstoffatom, eine Alkylgruppe mit 1 bis 5 Kohlenstoffatomen, eine Cycloalkylgruppe mit 3 bis 8 Kohlenstoffatomen oder einen Phenylrest

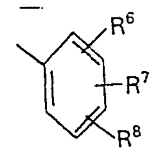

$R^{11}$ eine Alkylgruppe mit 1 bis 5 Kohlenstoffatomen, eine Cycloalkylgruppe mit 3 bis 8 Kohlenstoffatomen, eine Cycloalkylalkylgruppe mit 3 bis 8 Kohlenstoffatomen in der Cycloalkylgruppe und 1 bis 3 Kohlenstoffatomen in der Alkylgruppe, eine Phenylgruppe

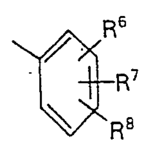

oder eine Phenylalkylgruppe mit 1 bis 3 Kohlenstoffatomen in der Alkylgruppe und mit dem Phenylrest

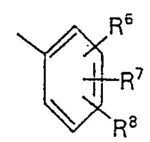

bedeutet oder

$R^{10}$ und $R^{11}$ gemeinsam eine Alkylengruppe mit 4 bis 7 Kohlenstoffatomen oder

$R^9$, $R^{10}$ und $R^{11}$ unter Einschluss des Kohlenstoffatoms, an das sie gebunden sind, einen Adamantylrest bedeuten,

sowie ihre Salze mit anorganischen und organischen Basen.

Anspruch 2

N-substituierte ω-Aminoalkanoyl-ω-aminoalkansäuren nach Anspruch 1, gekennzeichnet durch die allgemeine Formel I*

$$R^{1*}-\underset{\substack{|\\ R^{2*}}}{N}-A^{*}-CO-\underset{\substack{|\\ R^{3*}}}{N}-B^{*}-COOH \qquad (I^{*}),$$

worin

$R^{1*}$ einen Alkanoylrest oder Alkenoylrest mit 2 bis 5 Kohlenstoffatomen, einen Furoylrest oder einen Benzoylrest

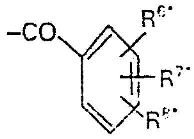

bedeutet,

$R^{2*}$ ein Wasserstoffatom, eine substituierte Niedrigalkylgruppe $-C(R^{9*})(R^{10*})(R^{11*})$ oder eine Phenylgruppe

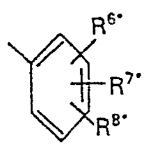

bedeutet,

$R^{3*}$ ein Wasserstoffatom oder eine gegebenenfalls substituierte Niedrigalkylgruppe bedeutet, wobei $R^{2*}$ und $R^{3*}$ nicht gleichzeitig ein Wasserstoffatom und $R^{2*}$ und $R^{3*}$ nicht gleichzeitig eine geradkettige Niedrigalkylgruppe darstellen,

A* eine $-(CH_2)_{m*}$-Gruppe bedeutet,

B* eine $-(CH_2)_{n*}$-Gruppe bedeutet,

m* und n* gleich oder verschieden sind und eine positive ganze Zahl von 1 bis 5 bedeuten,

$R^{6*}$, $R^{7*}$ und $R^{8*}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

$R^{9*}$ ein Wasserstoffatom, eine Alylgruppe mit 1 bis 4 Kohlenstoffatomen oder eine Ethinylgruppe bedeutet,

$R^{10*}$ ein Wasserstoffatom, eine Alkylgruppe mit 1 bis 4 Kohlenstoffatomen oder einen Phenylrest

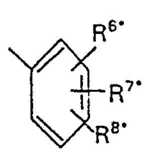

$R^{11*}$ eine Alkylgruppe mit 1 bis 5 Kohlenstoffatomen, einen Phenylrest

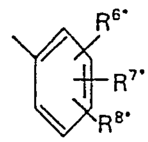

oder einen Benzylrest

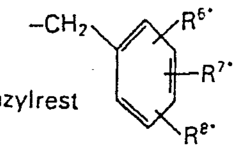

bedeutet oder

$R^{10*}$ und $R^{11*}$ gemeinsam eine Alkylengruppe mit 4 bis 7 Kohlenstoffatomen oder

$R^{9*}$, $R^{10*}$ und $R^{11*}$ unter Einschluss des benachbarten Kohlenstoffatoms einen Adamantyl-(1)-rest bedeuten,

und ihre Salze mit anorganischen und organischen Basen.

Anspruch 3

N-substituierte ω-Aminoalkanoyl-ω-aminoalkansäuren nach Anspruch 1, gekennzeichnet durch die allgemeine Formel I**

$$R^{1**}-\underset{\substack{|\\ R^{2**}}}{N}-A^{**}-CO-\underset{\substack{|\\ R^{3**}}}{N}-B^{**}-COOH \qquad (I^{**}),$$

worin

$R^{1**}$ einen Alkanoylrest oder Alkenoylrest mit 2 bis 5 Kohlenstoffatomen, einen Furoylrest oder einen Benzoylrest

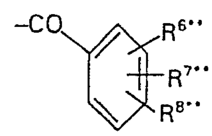

bedeutet,

$R^{2**}$ ein Wasserstoffatom oder eine Phenylgruppe

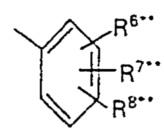

bedeutet,

$R^{3**}$ ein Wasserstoffatom oder eine Phenylgruppe

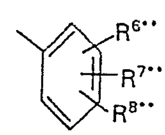

bedeutet, wobei $R^{2**}$ und $R^{3**}$ nicht gleichzeitig ein Wasserstoffatom darstellen,

A** eine $-(CH_2)_{m**}$-Gruppe bedeutet,

B** eine $-(CH_2)_{n**}$-Gruppe bedeutet,

m** und n** gleich oder verschieden sind und eine positive ganze Zahl von 1 bis 5 bedeuten,

$R^{6**}$, $R^{7**}$ und $R^{8**}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

und ihre Salze mit anorganischen und organischen Basen.

Anspruch 4

N-substituierte ω-Aminoalkanoyl-ω-aminoalkansäuren nach Anspruch 1, gekennzeichnet durch die allgemeine Formel I***

$$\begin{array}{l} R^{1***}\text{–}\underset{|}{N}\text{–}A^{***}\text{–CO–}\underset{|}{N}\text{–}B^{***}\text{–COOH} \\ \quad\; R^{2***} \qquad\qquad\quad R^{3***} \end{array} \qquad (I^{***}),$$

worin

$R^{1***}$ einen Alkanoyl- oder Alkenoylrest mit 2 bis 5 Kohlenstoffatomen, einen Furoylrest oder einen Benzoylrest

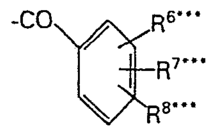

bedeutet,

$R^{2***}$ ein Wasserstoffatom oder einen Phenylrest

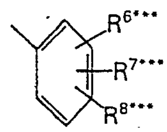

bedeutet,

$R^{3***}$ ein Wasserstoffatom, eine -C($R^{9***}$)($R^{10***}$)($R^{11***}$)-Gruppe oder eine durch $R^{6***}$, $R^{7***}$ und $R^{8***}$ substituierte Phenylgruppe bedeutet, wobei $R^{2***}$ und $R^{3***}$ nicht gleichzeitig ein Wasserstoffatom darstellen,

A*** eine $-(CH_2)_{m***}$-Gruppe oder eine -CH($R^{4***}$)-Gruppe bedeutet,

B*** eine $-(CH_2)_{n***}$-Gruppe oder eine -CH($R^{5***}$)-Gruppe bedeutet, wobei

A*** und B*** nicht gleichzeitig eine geradkettige Alkylengruppe darstellen,

m*** und n*** gleich oder verschieden sind und eine positive ganze Zahl von 1 bis 5 bedeuten,

$R^{4***}$ und $R^{5***}$ gleich oder verschieden sind und eine Methylgruppe, eine Benzylgruppe, eine Hydroxymethylgruppe oder eine 2-Methylmercaptoethylgruppe bedeuten, oder (unter der Voraussetzung, dass $R^2$ kein Wasserstoffatom darstellt),

$R^{3***}$ und $R^{5***}$ gemeinsam eine Trimethylengruppe bedeuten,

$R^{6***}$, $R^{7***}$ und $R^{8***}$ gleich oder verschieden sind und ein Wasserstoffatom, ein Halogenatom, eine Hydroxygruppe, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Aminogruppe, eine Nitrogruppe oder eine Trifluormethylgruppe bedeuten,

$R^{9***}$ ein Wasserstoffatom, eine Alkylgruppe mit 1 bis 3 Kohlenstoffatomen oder eine Ethinylgruppe bedeutet,

$R^{10***}$ ein Wasserstoffatom, eine Alkylgruppe mit 1 bis 3 Kohlenstoffatomen, eine Cycloalkylgruppe mit 3 bis 8 Kohlenstoffatomen oder einen durch $R^{6***}$, $R^{7***}$ und $R^{8***}$ substituierten Phenylrest bedeutet,

$R^{11***}$ eine Alkylgruppe mit 1 bis 3 Kohlenstoffatomen, eine Cycloalkylgruppe mit 3 bis 8 Kohlenstoffatomen, eine durch $R^{6***}$, $R^{7***}$ und $R^{8***}$ substituierte Phenylgruppe, eine durch $R^{6***}$, $R^{7***}$ und $R^{8***}$ substituierte Benzylgruppe bedeutet, oder

$R^{10***}$ und $R^{11***}$ gemeinsam eine Alkylengruppe mit 4 bis 7 Kohlenstoffatomen bedeuten,

und ihre Salze mit anorganischen und organischen Basen.

Anspruch 5

Arzneimittel, enthaltend eine oder mehrere Verbindungen nach einem oder mehreren der Ansprüche 1 bis 4 und/oder ihre pharmakologisch verträglichen Salze mit anorganischen oder organischen Basen.

Anspruch 6

Verfahren zur Herstellung von N-substituierten ω-Aminoalkanoyl-ω-aminoalkansäuren der allgemeinen Formel I

$$\begin{array}{l} R^{1}\text{–}\underset{|}{N}\text{–A–CO–}\underset{|}{N}\text{–B–COOH} \\ \quad R^{2} \qquad\qquad R^{3} \end{array} \qquad (I),$$

und ihren Salzen mit anorganischen und organischen Basen, dadurch gekennzeichnet, dass man eine ω-Aminoalkansäure der allgemeinen Formel II

$$\begin{array}{l} R^{1} \\ \quad \rangle N\text{–A–COOH} \\ R^{2} \end{array} \qquad (II),$$

gegebenenfalls nach Überführung in die entsprechenden Säurederivate mit einer ω-Aminoalkansäure der allgemeinen Formel III

$$R^3\text{–NH–B–COOH} \qquad (III),$$

umsetzt und gegebenenfalls in die Salze überführt, wobei in den Formeln I–III die Symbole $R^1$, $R^2$, $R^3$, A und B die in Anspruch 1 angegebenen Bedeutungen haben.

Anspruch 7

Verfahren zur Herstellung von N-substituierten ω-Aminoalkanoyl-ω-aminoalkansäuren der allgemeinen Formel I*

$$\begin{array}{l} R^{1*}\text{–}\underset{|}{N}\text{–}A^{*}\text{–CO–}\underset{|}{N}\text{–}B^{*}\text{–COOH} \\ \quad R^{2*} \qquad\qquad R^{3*} \end{array} \qquad (I^{*}),$$

und ihren Salzen mit anorganischen und organischen Basen, dadurch gekennzeichnet, dass man eine ω-Aminoalkansäure der allgemeinen Formel II*

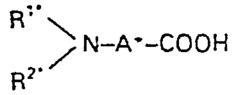

(II*),

gegebenenfalls nach Überführung in die entsprechenden Säurederivate, mit einer ω-Aminoalkansäure der allgemeinen Formel III*

$R^3$–NH–B*–COOH (III*),

umsetzt und gegebenenfalls in die Salze überführt, wobei in den Formeln I*–III* die Symbole $R^{1*}$, $R^{2*}$, $R^{3*}$, A* und B* die in Anspruch 2 angegebenen Bedeutungen haben.

Anspruch 8

Verfahren zur Herstellung von N-substituierten ω-Aminoalkanoyl-ω-aminoalkansäuren der allgemeinen Formel I**

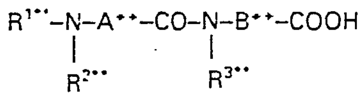

(I**),

und ihren Salzen mit anorganischen und organischen Basen, dadurch gekennzeichnet, dass man eine ω-Aminoalkansäure der allgemeinen Formel II**

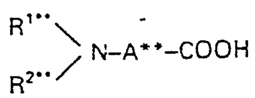

(II**),

gegebenenfalls nach Überführung in die entsprechenden Säurederivate, mit einer ω-Aminoalkansäure der allgemeinen Formel III**

$R^{3**}$–NH–B**–COOH (III**),

umsetzt und gegebenenfalls in die Salze überführt, wobei in den Formeln I**–III** die Symbole $R^{1**}$, $R^{2**}$, $R^{3**}$, A** und B** die in Anspruch 3 angegebenen Bedeutungen haben.

Anspruch 9

Verfahren zur Herstellung von N-substituierten ω-Aminoalkanoyl-ω-aminoalkansäuren der allgemeinen Formel I***

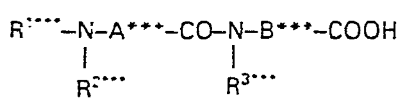

(I***),

und ihren Salzen mit anorganischen und organischen Basen, dadurch gekennzeichnet, dass man eine ω-Aminoalkansäure der allgemeinen Formel II***

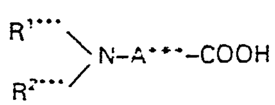

(II***),

gegebenenfalls nach Überführung in die entsprechenden Säurederivate, mit einer ω-Aminoalkansäure der allgemeinen Formel III***

$R^{3***}$–NH–B***–COOH (III***),

umsetzt und gegebenenfalls in die Salze überführt, wobei in den Formeln I***–III*** die Symbole $R^{1***}$, $R^{2***}$, $R^{3***}$, A*** und B*** die in Anspruch 4 angegebenen Bedeutungen haben.

Anspruch 10

Verbindungen gemäss einem oder mehreren der Ansprüche 1 bis 4 zur Behandlung oder Prophylaxe von Krankheiten, die auf Erkrankungen des Magens oder Darms oder auf Minderleistungen von Pankreas, Galle und oder Leber beruhen.

Claims

1. N-substituted ω-aminoalkanoyl-ω-aminoalkanoic acids of the general formula I

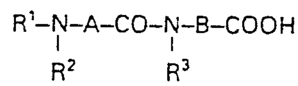

(I),

wherein

$R^1$ signifies an alkanoyl radical or alkenoyl radical with 2 to 5 carbon atoms, a furoyl or a benzoyl radical

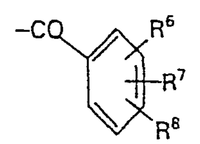

$R^2$ signifies a hydrogen atom, a $-C(R^9)(R^{10})(R^{11})$-group or a phenyl group

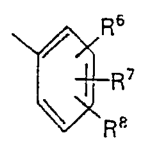

$R^3$ signifies a hydrogen atom, an alkyl radical with from 1 to 5 carbon atoms, a $-C(R^9)(R^{10})(R^{11})$-group or a phenyl group

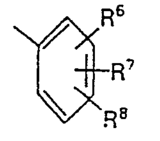

$R^2$ and $R^3$ not being at the same time a hydrogen atom or a straight alkyl radical,

A signifies a $-(CH_2)_m$-group or a $-CH(R^4)$-group,

B signifies a $-(CH_2)_n$-group or a $-CH(R^5)$-group,

m and n are the same or different and signify a positive whole number from 1 to 5,

$R^4$ and $R^5$ are the same or different and signify a methyl group, a benzyl group, a hydroxymethyl group, a 2-hydroxyethyl group, a methylthiomethyl group, or a 2-methylthioethyl group or (provided that $R^2$ is no hydrogen atom),

$R^3$ and $R^5$ together signify a trimethylene group,

$R^6$, $R^7$ and $R^8$ are the same or different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, an amino group, a nitro group or a trifluoromethyl group,

$R^9$ signifies a hydrogen atom, an alkyl group with 1 to 5 carbon atoms, an alkenyl group or an alkinyl group with 2 to 5 carbon atoms,

$R^{10}$ signifies a hydrogen atom, an alkyl group with 1 to 5 carbon atoms, a cycloalkyl group with 3 to 8 carbon atoms or a phenyl radical

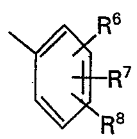

$R^{11}$ signifies an alkyl group with 1 to 5 carbon atoms, a cycloalkyl group with 3 to 8 carbon atoms, a cycloalkylalkyl group with 3 to 8 carbon atoms in the cycloalkyl moiety and 1 to 3 carbon atoms in the alkyl moiety, a phenyl radical

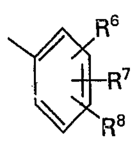

or a phenylalkyl group with 1 to 3 carbon atoms in the alkyl moiety and with the phenyl radical

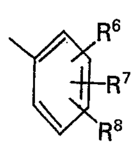

or

$R^{10}$ and $R^{11}$ together signify an alkylene group with 4 to 7 carbon atoms or

$R^9$, $R^{10}$ and $R^{11}$ with the inclusion of the neighbouring carbon atom, to which they are bound, signify an adamantyl radical,

and their salts of inorganic and organic bases.

2. N-substituted ω-aminoalkanoyl-ω-aminoalkanoic acids according to claim 1, characterized by the general formula I*

$$R^{1*}-\underset{\underset{R^{2*}}{|}}{N}-A^*-CO-\underset{\underset{R^{3*}}{|}}{N}-B^*-COOH \qquad (I^*),$$

in which

$R^{1*}$ signifies an alkanoyl radical or alkenoyl radical with 2 to 5 carbon atoms, a furoyl radical or a benzoyl radical

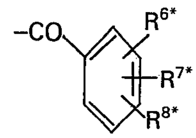

$R^{2*}$ signifies a hydrogen atom, a substituted lower alkyl group $-C(R^{9*})(R^{10*})(R^{11*})$ or a phenyl group

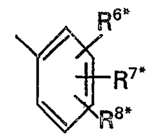

$R^{3*}$ signifies a hydrogen atom or an optionally substituted lower alkyl group, in which $R^{2*}$ and $R^{3*}$ do not simultaneously signify a hydrogen atom and $R^{2*}$ and $R^{3*}$ do not simultaneously represent a straight-chained lower alkyl group,

A* signifies a $-(CH_2)_{m*}$-group,

B* signifies a $-(CH_2)_{n*}$-group,

m* and n* are the same or different and signify a positive whole number from 1 to 5,

$R^{6*}$, $R^{7*}$ and $R^{8*}$ are the same of different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, an amino group, a nitro group or a trifluoromethyl group,

$R^{9*}$ signifies a hydrogen atom, an alkyl group with 1 to 4 carbon atoms or an ethinyl group,

$R^{10*}$ signifies a hydrogen atom, an alkyl group with 1 to 4 carbon atoms or a phenyl radical

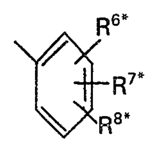

$R^{11*}$ signifies an alkyl group with 1 to 5 carbon atoms, a phenyl radical

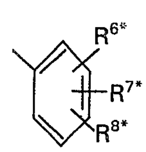

or a benzyl radical

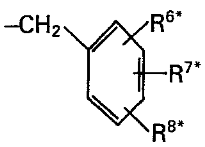

or

$R^{10*}$ and $R^{11*}$ together signify an alkylene group with 4 to 7 carbon atoms or

$R^{9*}$, $R^{10*}$ and $R^{11*}$ with the inclusion of the neighbouring carbon atom signify an adamantyl-(1) radical,

and their salts of inorganic and organic bases.

3. N-substituted ω-aminoalkanoyl-ω-aminoalkanoic acids according to claim 1, characterized by the general formula I**

$$R^{1**}\text{-}\underset{R^{2**}}{\underset{|}{N}}\text{-}A^{**}\text{-}CO\text{-}\underset{R^{3**}}{\underset{|}{N}}\text{-}B^{**}\text{-}COOH \quad (I^{**}),$$

in which

$R^{1**}$ signifies an alkanoyl radical or alkenoyl radical with 2 to 5 carbon atoms, a furoyl radical or a benzoyl radical

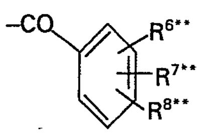

$R^{2**}$ signifies a hydrogen atom or a phenyl group

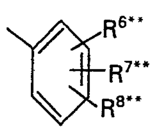

$R^{3**}$ signifies a hydrogen atom or a phenyl group

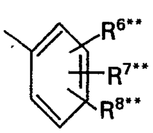

in which $R^{2**}$ and $R^{3**}$ cannot at the same time represent a hydrogen atom,

A** signifies a $-(CH_2)_{m**}$-group,

B** signifies a $-(CH_2)_{n**}$-group,

m** and n** are the same or different and signify a positive whole number from 1 to 5,

$R^{6**}$, $R^{7**}$ and $R^{8**}$ are the same or different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, an amino group, a nitro group or a trifluoromethyl group,

and their salts of inorganic and organic bases.

4. N-substituted ω-aminoalkanoyl-ω-aminoalkanoic acids according to claim 1, characterized by the general formula I***

$$R^{1***}\text{-}\underset{R^{2***}}{\underset{|}{N}}\text{-}A^{***}\text{-}CO\text{-}\underset{R^{3***}}{\underset{|}{N}}\text{-}B^{***}\text{-}COOH \quad (I^{***}),$$

in which

$R^{1***}$ signifies an alkanoyl or alkenoyl radical with 2 to 5 carbon atoms, a furoyl radical or a benzoyl radical

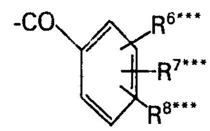

$R^{2***}$ signifies a hydrogen atom or a phenyl radical

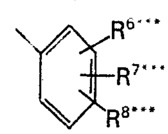

$R^{3***}$ signifies a hydrogen atom, a $-C(R^{9***})(R^{10***})(R^{11***})$-group or a phenyl group substituted with $R^{6***}$, $R^{7***}$ and $R^{8***}$, in which $R^{2***}$ and $R^{3***}$ do not at the same time represent a hydrogen atom.

A*** signifies a $-(CH_2)_{m***}$-group or a $-CH(R^{4***})$-group,

B*** signifies a $-(CH_2)_{n***}$-group or a $-CH(R^{5***})$-group,

in which A*** and B*** do not at the same time represent a straight-chain alkylene group,

m*** and n*** are the same or different and signify a positive whole number from 1 to 5.

$R^{4***}$ and $R^{5***}$ are the same or different and signify a methyl group, a benzyl group, a hydroxymethyl group or a 2-methylmercaptoethyl group, or (provided that $R^{4***}$ is no hydrogen atom).

$R^{3***}$ and $R^{5***}$ together signify a trimethylene group,

$R^{6***}$, $R^{7***}$ and $R^{8***}$ are the same or different and signify a hydrogen atom, a halogen atom, a hydroxy group, an alkyl group with 1 to 4 carbon atoms, an alkoxy group with 1 to 4 carbon atoms, an alkanoyloxy group with 2 to 5 carbon atoms, an amino group, a nitro group or a trifluoromethyl group,

$R^{9***}$ signifies a hydrogen atom, an alkyl group with 1 to 3 carbon atoms or an ethinyl group,

$R^{10***}$ signifies a hydrogen atom, an alkyl group with 1 to 3 carbon atoms, a cycloalkyl group with 3 to 8 carbon atoms or a phenyl radical substituted with $R^{6***}$, $R^{7***}$ and $R^{8***}$,

$R^{11***}$ signifies an alkyl group with 1 to 3 carbon atoms, a cycloalkyl group with 3 to 8 carbon atoms , a phenyl group substituted with $R^{6***}$, $R^{7***}$ and $R^{8***}$, a benzyl group substituted with $R^{6***}$, $R^{7***}$ an $R^{8***}$, or

$R^{10***}$ and $R^{11***}$ together signify an alkylene group with 4 to 7 carbon atoms, and their salts of inorganic and organic bases.

5. Medicaments containing one or more compounds in accordance with one or more of the claims 1 to 4 and/or their pharmacologically compatible salts of inorganic or organic bases.

6. Processes for the production of N-substituted ω-aminoalkanoyl-ω-aminoalkanoic acids of the general formula I

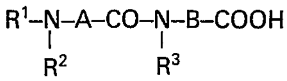 (I),

and their salts of inorganic and organic bases, characterised by the fact that an ω-aminoalkanoic acid of the general formula II

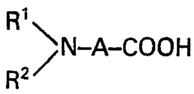 (II),

if desired after conversion into a corresponding acid derivative, is reacted with an ω-aminoalkanoic acid of the general formula III

$R^3$–NH–B–COOH (III),

and if desired is converted into the salts, the symbols $R^1$, $R^2$, $R^3$, A and B of the formulae I to III having the meaning given in claim 1.

7. Process for the production of N-substituted ω-aminoalkanoyl-ω-aminoalkanoic acids of the general formula I*

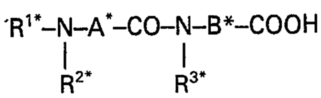 (I*),

and their salts of inorganic and organic bases, characterised by the fact that an ω-aminoalkanoic acid of the general formula II*

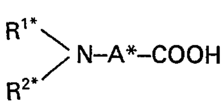 (II*),

if desired after conversion into a corresponding acid derivative, is reacted with an ω-aminoalkanoic acid of the general formula III*

$R^{3*}$–NH–B*–COOH (III*),

and if desired is converted into the salts, the symbols $R^{1*}$, $R^{2*}$, $R^{3*}$, A* and B* of the formulae I* to III* having the meaning given in claim 2.

8. Process for the production of N-substituted ω-aminoalkanoyl-ω-aminoakanoic acids of the general formula I**

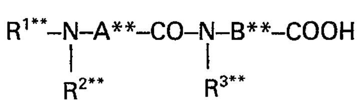 (I**),

and their salts of inorganic and organic bases, characterised by the fact that an ω-aminoalkanoic acid of the general formula II**

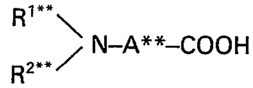 (II**),

if desired after conversion into a corresponding acid derivative, is reacted with an ω-aminoalkanoic acid of the general formula III**

$R^{3**}$–NH–B**–COOH (III**),

and if desired is converted into the salts, the symbols $R^{1**}$, $R^{2**}$, $R^{3**}$, A** and B** of the formulae I** to III** having the meaning given in claim 3.

9. Process for the production of N-substituted ω-aminoalkanoyl-ω-aminoalkanoic acids of the general formula I***

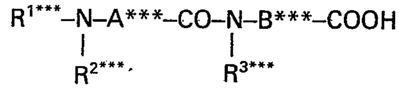 (I***),

and their salts of inorganic and organic bases, characterised by the fact that an ω-aminoalkanoic acid of the general formula II***

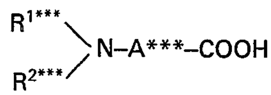 (II***),

if desired after conversion into a corresponding acid derivative, is reacted with an ω-aminoalkanoic acid of the general formula III***

$R^{3***}$–NH–B***–COOH (III***),

and if desired is converted into the salts, the symbols $R^{1***}$, $R^{2***}$, $R^{3***}$, A*** and B*** of the formulae I*** to III*** having the meaning given in claim 4.

10. Compounds in accordance with one or more of the claims 1 to 4 for the treatment and prophylaxis of diseases which are attributable to disorders of the stomach or intestine or to reduces performances of the pancreas, bile and/or liver.

**Revendications**

1. Acides ω-aminoalkanoyl-ω-aminoalkancarboxyliques N-substitués de la formula générale I:

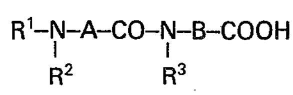 (I),

dans laquelle

$R^1$ représente un reste alkanoyle ou alkénoyle comportant de 2 à 5 atomes de carbone, un reste furoyle ou un reste benzoyle

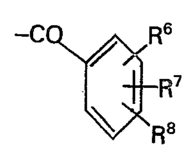

$R^2$ représente un atome d'hydrogène, un groupe $-C(R^9)(R^{10})(R^{11})$ ou un groupe phényle

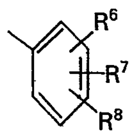

$R^3$ représente un atome d'hydrogène, un groupe alkyle comportant de 1 à 5 atomes de carbone, le groupe $-C(R^9)(R^{10})(R^{11})$ ou un groupe phényle

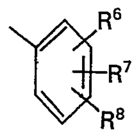

$R^2$ et $R^3$ ne pouvant pas représenter simultanément un atome d'hydrogène et $R^2$ et $R^3$ ne pouvant pas représenter simultanément un groupe alkyle à chaîne linéaire,

A représente un groupe $-(CH_2)_m$ ou un groupe $-CH(R^4)$,

B représente un groupe $-(CH_2)_n$ ou un groupe $-CH(R^5)$,

m et n sont identiques ou différents et représentent un nombre entier positif de 1 à 5,

$R^4$ et $R^5$ sont identiques ou différents et représentent un groupe méthyle, un groupe benzyle, un groupe hydroxyméthyle, un groupe 2-hydroxyéthyle, un groupe méthylmercaptométhyle ou un groupe 2-méthylmercaptoéthyle ou (à condition que $R^2$ ne représente pas un atome d'hydrogène)

$R^3$ et $R^5$ forment ensemble un groupe triméthylène,

$R^6$, $R^7$ et $R^8$ sont identiques ou différents et représentent un atome d'hydrogène, un atome d'halogène, un groupe hydroxy, un groupe alkyle comportant de 1 à 4 atomes de carbone, un groupe alkoxy comportant de 1 à 4 atomes de carbone, un groupe alkanoyloxy comportant de 2 a 5 atomes de carbone, un groupe amino, un groupe nitro ou un groupe trifluorométhyle,

$R^9$ représente un atome d'hydrogène, un groupe alkyle comportant de 1 à 5 atomes de carbone, un groupe alkényle ou un groupe alkinyle comportant de 2 à 5 atomes de carbone,

$R^{10}$ représente un atome d'hydrogène, un groupe alkyle comportant de 1 à 5 atomes de carbone, un groupe cycloalkyle comportant de 3 à 8 atomes de carbone ou le reste phényle

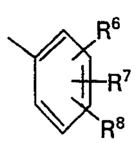

$R^{11}$ représente un groupe alkyle comportant de 1 à 5 atomes de carbone, un groupe cycloalkyle comportant de 3 à 8 atomes de carbone, un groupe cycloalkylalkyle comportant de 3 à 8 atomes de carbone dans le groupe cycloalkyle et de 1 à 3 atomes de carbone dans le groupe alkyle, un groupe phényle

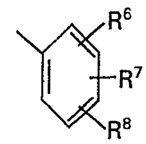

ou un groupe phénylalkyle comportant de 1 à 3 atomes de carbone dans le groupe alkyle et avec le groupe phényle

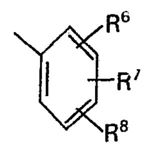

ou

$R^{10}$ et $R^{11}$ représentent ensemble un groupe alkylène comportant de 4 à 7 atomes de carbone ou

$R^9$, $R^{10}$ et $R^{11}$ en englobant l'atome de carbone auquel ils sont liés, forment un reste adamantyle,

ainsi que les sels de ces composés des bases inorganiques ou organiques.

2. Acides ω-aminoalkanoyl-ω-aminoalkancarboxyliques N-substitués selon la revendication 1, caractérisés en ce qu'ils répondent à la formule générale I*:

$$R^{1*}-\underset{\underset{R^{2*}}{|}}{N}-A^*-CO-\underset{\underset{R^{3*}}{|}}{N}-B^*-COOH \qquad (I^*),$$

dans laquelle

$R^{1*}$ représente un reste alkanoyle ou alkénoyle comportant de 2 à 5 atomes de carbone, un reste furoyle ou un reste benzoyle

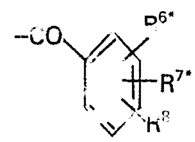

$R^{2*}$ représente un atome d'hydrogène, un groupe alkyle inférieur substitué $-C(R^{9*})(R^{10*})(R^{11*})$ ou un groupe phényle

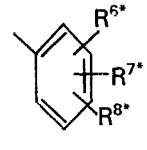

$R^{3*}$ représente un atome d'hydrogène ou un groupe alkyle inférieur éventuellement substitué,

$R^{2*}$ et $R^{3*}$ ne pouvant pas représenter simultanément un atome d'hydrogène et $R^{2*}$ et

$R^{3*}$ ne pouvant pas représenter simultanément un groupe alkyle inférieur à chaîne linéaire,

A* représente le groupe $-(CH_2)_{m*}$,

B* représente le groupe $-(CH_2)_{n*}$,

m* et n* sont identiques ou différents et représentent un nombre entier positif de 1 à 5,

$R^{6*}$, $R^{7*}$ et $R^{8*}$ sont identiques ou différents et représentent un atome d'hydrogène, un atome d'halogène, un groupe hydroxy, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro ou un groupe trifluorométhyle,

$R^{9*}$ représente un atome d'hydrogène, un groupe alkyle comportant de 1 à 4 atomes de carbone ou un groupe éthinyle,

$R^{10*}$ représente un atome d'hydogène, un groupe alkyle comportant de 1 à 4 atomes de carbone ou le reste phényle

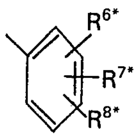

$R^{11*}$ représente un groupe alkyle comportant de 1 à 5 atomes de carbone, un reste phényle

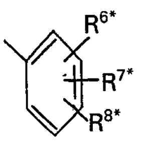 ou un reste benzyle 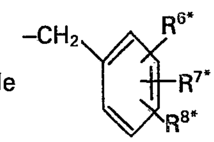

ou

$R^{10*}$ et $R^{11*}$ représentent ensemble un groupe alkylène comportant de 4 à 7 atomes de carbone ou encore

$R^{9*}$, $R^{10*}$ et $R^{11*}$, en englobant l'atome de carbone vicinal, forment un reste adamantyle-(1),

ainsi que les sels de ces composés avec des bases inorganiques ou organiques.

3. Acides ω-aminoalkanoyl-ω-aminoalkancarboxyliques N-substitués selon la revendication 1, charactérisés en ce qu'ils répondent à la formule générale I**

$$R^{1**}-\underset{\large R^{2**}}{\underset{|}{N}}-A^{**}-CO-\underset{\large R^{3**}}{\underset{|}{N}}-B^{**}-COOH \qquad (I^{**}),$$

dans laquelle

$R^{1**}$ représente un reste alkanoyle ou un reste alkénoyle comportant de 2 à 5 atomes de carbone, un reste furoyle ou un reste benzoyle

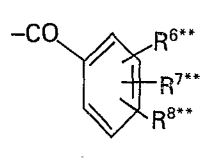

$R^{2**}$ représente un atome d'hydrogène ou un groupe phényle

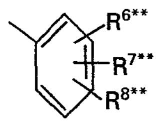

$R^{3**}$ représente un atome d'hydrogène ou un groupe phényle

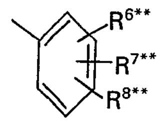

$R^{2**}$ et $R^{3**}$ ne représentant pas simultanément un atome d'hydrogène,

A** représente un groupe $-(CH_2)_{m**}$,

B** représente un groupe $-(CH_2)_{n**}$,

m** et n** étant identiques ou différents et représentant un nombre entier positif de 1 à 5,

$R^{6**}$, $R^{7**}$ et $R^{8**}$ sont identiques ou différents et représentent un atome d'hydrogène, un atome d'halogène, un groupe hydroxy, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro ou un groupe trifluorométhyle,

ainsi que les sels de ces composés avec des bases inorganiques ou organiques.

4. Acides ω-aminoalkanoyl-ω-aminoalkancarboxyliques N-substitués selon la revendication 1, charactérisés en ce qu'ils répondent à la formule générale I***

$$R^{1***}-\underset{\large R^{2***}}{\underset{|}{N}}-A^{***}-CO-\underset{\large R^{3***}}{\underset{|}{N}}-B^{***}-COOH \qquad (I^{***}),$$

dans laquelle

$R^{1***}$ représente un reste alkanoyle ou alkénoyle comportant de 2 à 5 atomes de carbone, un reste furoyle ou un reste benzoyle

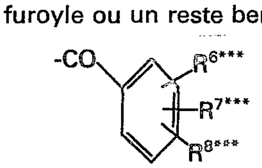

$R^{2***}$ représente un atome d'hydrogène ou un reste phényle

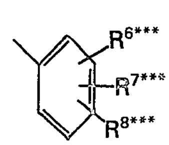

$R^{3***}$ représente un atome d'hydrogène, un groupe $-C(R^{9***})(R^{10***})(R^{11***})$ ou un groupe phényle substitué par $R^{6***}$, $R^{7***}$ et $R^{8***}$, $R^{2***}$ et $R^{3***}$ ne pouvant pas représenter simultanément un atome d'hydrogène,

A*** représente un groupe $-(CH_2)_{m***}$ ou un groupe $-CH(R^{4***})$,

B*** représente un groupe $-(CH_2)_{n***}$ ou un groupe $-CH(R^{5***})$,

A*** et B*** ne pouvant pas représenter simultanément un groupe alkylène linéaire,

m*** et n*** sont identiques ou différents et représentent un nombre entier positif de 1 à 5,

$R^{4***}$ et $R^{5***}$ sont identiques ou différents et représentent un groupe méthyle, un groupe benzyle, un groupe hydroxyméthyle ou un groupe 2-méthylmercaptoéthyle, ou (à condition que $R^{2***}$ ne représente pas un atome d'hydrogène),

$R^{3***}$ et $R^{5***}$ forment ensemble un groupe triméthylène,

$R^{6***}$, $R^{7***}$ et $R^{8***}$ sont identiques ou différents et représentent un atome d'hydrogène, un atome d'halogène, un groupe hydroxy, un groupe alkyle comportant de 1 à 4 atomes de carbone, un groupe alkoxy comportant de 1 à 4 atome de carbone, un groupe alkanoyloxy comportant de 2 à 5 atomes de carbone, un groupe amino, un groupe nitro ou un groupe trifluorométhyle,

$R^{9***}$ représente un atome d'hydrogène, un groupe alkyle comportant de 1 à 3 atomes de carbone ou un groupe éthinyle,

$R^{10***}$ représente un atome d'hydrogène, un groupe alkyle comportant de 1 à 3 atomes de carbone, un groupe cycloalkyle comportant de 3 à 8 atomes de carbone ou un reste phényle substitué par $R^{6***}$, $R^{7***}$ et $R^{8***}$,

$R^{11***}$ représente un groupe alkyle comportant de 1 à 3 atomes de carbone, un groupe cycloalkyle comportant de 3 à 8 atomes de carbone, un groupe phényle substitué par $R^{6***}$, $R^{7***}$ et $R^{8***}$, un groupe benzyle substitué par $R^{6***}$, $R^{7***}$ et $R^{8***}$, ou

$R^{10***}$ et $R^{11***}$ forment ensemble un groupe alkylène comportant de 4 à 7 atomes de carbone,

ainsi que les sels de ces composés avec des bases inorganiques ou organiques.

5. Médicaments contenant un ou plusieurs composés selon une ou plusieurs des revendications 1 à 4 et/ou leurs sels pharmacologiquement acceptables avec des bases inorganiques ou organiques.

6. Procédé pour la préparation d'acides ω-aminoalkanoyl-ω-aminoalkancarboxyliques N-substitués de la formule générale I

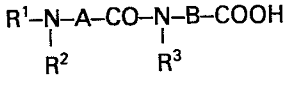
(I),

ainsi que de leurs sels avec des bases inorganiques et organiques, charactérisé en ce qu'un acide ω-aminoalkancarboxylique de la formule générale II

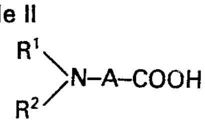
(II),

éventuellement après conversion en dérivés d'acides correspondants, est mis en réaction avec un acide ω-aminoalkancarboxylique de la formule générale III

$R^3$–NH–B–COOH (III),

et qu'on transforme éventuellement le composé en un sel, les symboles $R^1$, $R^2$, $R^3$, A et B dans les formules I à III ayant les significations identiquées dans la revendication 1.

7. Procédé pour la préparation d'acides ω-aminoalkanoyl-ω-aminoalkancarboxyliques N-substitués de la formule générale I*:

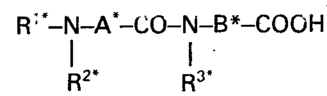
(I*),

ainsi que de leurs sels avec des bases inorganiques et organiques, charactérisé en ce qu'un acide ω-aminoalkanoïque de la formule générale II*

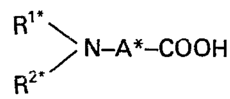
(II*),

éventuellement après conversion en dérivés d'acides correspondants, est mis en réaction avec un acide ω-aminoalkancarboxylique de la formule générale III*

$R^{3*}$–NH–B*–COOH (III*),

et qu'on transforme eventuellement le composé en un sel, les symboles $R^{1*}$, $R^{2*}$, $R^{3*}$, A* et B* dans les formules I* à III* ayant les significations indiquees dans la revendication 2.

8. Procédé pour la préparation d'acides ω-aminoalkanoyl-ω-aminoalkancarboxyliques N-substitués de la formule générale I**

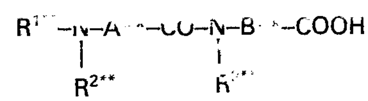
(I**),

ainsi que de leurs sels avec des bases inorganiques et organiques, charactérisé en ce qu'un acide ω-aminoalkancarboxylique de la formule générale II**

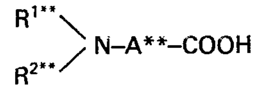
(II**),

éventuellement après conversion en dérivés d'acides correspondants, est mis en réaction avec un acide ω-aminoalkancarboxylique de la formule générale III**

$R^{3**}$–NH–B**–COOH (III**),

et qu'on transforme éventuellement le composé en un sel, les symboles $R^{1**}$, $R^{2**}$, $R^{3**}$, A** et B**

dans les formules I** à III** ayant les significations indiquées dans la revendication 3.

9. Procédé pour la préparation d'acides ω-aminoalkanoyl-ω-aminoalkancarboxyliques N-substitués de la formule générale I***

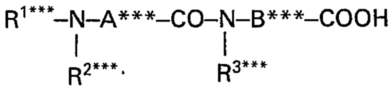

(I***),

ainsi que de leurs sels avec des bases inorganiques et organiques, charactérisé en ce qu'un acide ω-aminoalkancarboxylique de la formule générale II***

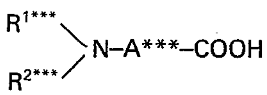

(II***),

éventuellement après conversion en dérivés d'acides correspondants, est mis en réaction avec un acide ω-aminoalkancarboxylique de la formule générale III***

$R^{3***}$–NH–B***–COOH (III***),

et qu'on transforme éventuellement le composé en un sel, les symboles $R^{1***}$, $R^{2***}$, $R^{3***}$, A*** et B*** dans les formules I*** à III*** ayant les significations indiquées dans la revendication 4.

10. Composés selon une ou plusieurs des revendications 1 à 4 pour le traitement et la prophylaxie de maladies dues à des affections de l'estomac ou de l'intestine ou encore à un rendement moins bon du pancréas, de la bile et/ou du foie.